(19) Europäisches Patentamt / European Patent Office / Office européen des brevets

(11) **EP 2 944 266 A1**

(12) **EUROPEAN PATENT APPLICATION**
published in accordance with Art. 153(4) EPC

(43) Date of publication:
**18.11.2015 Bulletin 2015/47**

(51) Int Cl.:
***A61B 8/00*** *(2006.01)*

(21) Application number: **14737504.2**

(22) Date of filing: **10.01.2014**

(86) International application number:
**PCT/JP2014/050344**

(87) International publication number:
**WO 2014/109392 (17.07.2014 Gazette 2014/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.01.2013   JP 2013003863**

(71) Applicant: **Hitachi Aloka Medical, Ltd.**
**Tokyo 181-8622 (JP)**

(72) Inventors:
• **IKEDA, Teiichiro**
  **Tokyo 100-8280 (JP)**
• **MASUZAWA, Hiroshi**
  **Tokyo 100-8280 (JP)**
• **TAKANO, Shinta**
  **Tokyo 100-8280 (JP)**
• **ISHIHARA, Chizue**
  **Tokyo 100-8280 (JP)**

(74) Representative: **MERH-IP**
**Matias Erny Reichl Hoffmann**
**Paul-Heyse-Strasse 29**
**80336 München (DE)**

(54) **ULTRASONIC IMAGING DEVICE**

(57)     Provided is an ultrasound imaging apparatus which can compensate deterioration in the image quality resulting from heterogeneity of a test object medium.

A reception beamformer 108 performs synthesizing after performing phasing processing of a signal received by an ultrasound element array 105 for each of two or more steering directions. The two or more steering directions are instructed by a steering direction instruction unit 112. The two or more steering directions include two directions each of which forms predetermined angles on the right and left with respect to a direction of a reception focal point along the ultrasound element array in an array direction. The predetermined angles are preferably null angles.

Fig. 1

EP 2 944 266 A1

## Description

Technical Field

The present invention relates to an ultrasound imaging technology for capturing an image inside a test object by using ultrasound waves.

Background Art

Ultrasound imaging technology is a technology for non-invasively imaging the inside of a test object including a human body by using ultrasound waves (a sound wave not intended to be heard, sound waves at a high frequency equal to or greater than 20 kHz, generally). As an example, a medical ultrasound imaging apparatus will be described simply. An ultrasound probe transmits an ultrasound wave into the body of a patient and receives an echo signal reflected from the inside of the patient's body. The reception signal is subjected to signal processing in any one of or both the ultrasound probe and an ultrasound imaging apparatus main body. Thereafter, the reception signal is transferred to an image display unit, and an ultrasound image is displayed. More specifically, for example, a signal of a transmission beam is generated by a transmission beamformer in the ultrasound imaging apparatus main body, and then, the signal passes through a transmission/reception separation circuit (T/R), thereby being sent to the ultrasound probe. The ultrasound probe transmits ultrasound waves. After receiving an echo signal from the inside of a human body, the ultrasound probe transmits a signal to the imaging apparatus main body. In the imaging apparatus main body, the reception signal passes through the transmission/reception separation circuit again and is subjected to phasing processing by a reception beamformer, thereby being transmitted to an image processing unit. In the image processing unit, various types of image processing such as diverse filtering and scan converting are executed. Eventually, an ultrasound image is displayed by the image display unit.

In this manner, a general ultrasound diagnostic apparatus is configured by three technologies such as transmission beamforming, reception beamforming, and the back-end image processing. Particularly, since the beamformer performs signal processing at the RF (high frequency) level during transmission and reception, algorithm of the beamformer or mounting architecture decides the basic image quality of an ultrasound image. Therefore, the beamformer is a key unit in the apparatus.

The reception beamformer applies a delay time in which a delay amount is distributed in a concave surface-type in accordance with a relationship between a focal position and an element position with respect to each reception signal (reception data) of multiple elements configuring the ultrasound probe. The reception beamformer sets a focal point to be focused on a certain point in a virtual space and performs adding of reception signal data. This method is called phasing performed by a delay adding method. In the delay adding method, reception data received by the multiple elements of the ultrasound diagnostic apparatus is multiplied by a fixed weight vector which is saved in a diagnostic apparatus. Then, adding is performed after performing apodization. The method is applied to not only the reception beamformer but is applied similarly to the transmission beamformer.

Meanwhile, it is understood that there is a limitation on azimuthal direction resolution as a basic problem of the ultrasound imaging apparatus. Since transmission and reception of an ultrasound wave are performed by an array which has a finite open caliber, there is an occurrence of an influence to diffraction at the edge of the opening portion. It is possible to infinitely improve the resolution in accordance with an improved depth direction if an infinitely long array can be prepared. However, realistically, due to the physical limitation on apparatus design such as the length of a transmission/reception array, improvement of azimuthal direction resolution has been restricted. Recently, an attempt attracting attention thereto has been made. That is, the aforementioned fixed weight vector which is used for performing delaying during delay adding of the beamformer is adaptively varied with respect to every single item of transmission/reception data in chronological order, thereby acquiring a more accurate ultrasound image. Accordingly, it is possible to particularly improve azimuthal direction resolution which has been an essential problem in the beamforming technology.

Particularly in recent years, reports on a technology for improving azimuthal direction resolution have begun to be made. In the technology, technologies of adaptive signal processing including the MVDR method (minimum variance distortionless response; the capon method) that have been developed in the mobile communication field are applied to a beamformer of reception data. The aforementioned adaptive techniques are realized by adaptively varying a complex component in the weight vector for performing delay adding, based on a correlation matrix of reception data. In other words, in the related art, the weight vector used to be a fixed value, but in the adaptive technique, the weight vector is obtained through computation by using a reception signal for each sample point in a temporal direction of the reception signal, and the obtained weight vector is multiplied by the reception signal.

In adaptive signal processing, similar to the delay adding method in the related art, there is a problem of distortion in ultrasound wave-front resulting from distribution of heterogenous sound velocities in a medium, and a randomly scattered micro body. In adaptive signal processing, the focal point of the reception beamformer set in the apparatus is set while assuming that the medium is uniform in sound velocity and is homogeneous (homogeneity). Therefore, when there is a presence of distortion in sound wave propagation, the distortion may lead to blurring of an image or image formation at a place different from the actual position. In the delay adding method in the related art, correction of wave-front distortion has been a problem for a long time. Accordingly, an aberration correction technology utilizing cross-correlation processing is examined, and similarly in the adaptive beamformer, significantly varying the image quality of an ultrasound image due to heterogeneity inside the medium has become a problem.

As the related art of the beamformer, for example, PTL 1 discloses a technology of a delay adding beamformer which utilizes a fixed null direction.

According to the technology in PTL 1, in order to decrease an artifact due to a side lobe, a main lobe is oriented in a predetermined direction of the target from which information is intended to be acquired so as to generate a first reception signal. Then, the null direction is oriented in the predetermined direction, thereby generating a second reception signal. The second reception signal is an artifact (noise) signal which negligibly includes information regarding a predetermined direction of the target. Therefore, the second reception signal is subtracted from the first reception signal. Thus, a useful signal component in an ultrasound image can be prevented from lacking, and the artifact can be decreased in the first reception signal (particularly, in paragraph 0060 in PTL 1).

Citation List

Patent Literature

PTL 1: JP-A-2010-158374

Summary of Invention

Technical Problem

Since an actual test object medium is heterogenous, a transmitted ultrasound signal is reflected by a focal point and scatters over the periphery of the focal point. Accordingly, target information at the focal point also exists on the periphery of the focal point. Since an adaptive beamformer has sharp directivity, the ultrasound signal existing on the periphery of the focal point cannot be acquired, thereby leading to a problem of deterioration in the image quality of an ultrasound image.

In addition, due to the heterogenous test object medium, there is an occurrence of wave-front distortion resulting from non-uniformity of sound velocity and the like, and the wave-front distortion causes a problem that a reflected wave from the medium on the periphery of the focal point may be mixed into a reception sound wave. Since the reflected wave from the medium on the periphery thereof is a noise signal (correlative noise) which is correlated to a signal from the focal point, it is difficult to be eliminated through a general noise decreasing technology. The correlative noise causes deterioration in the image quality of an ultrasound image.

An object of the present invention is to provide an ultrasound imaging apparatus which can compensate deterioration in the image quality resulting from heterogeneity of a test object medium.

Solution to Problem

A reception beamformer of an ultrasound imaging apparatus according to the present invention includes a phasing synthesizing unit which performs synthesizing after performing phasing processing of the signal received by the ultrasound element array for each of two or more steering directions and a steering direction instruction unit which instructs the phasing synthesizing unit to perform phasing in the two or more steering directions. The two or more steering directions include at least two directions other than a direction of a reception focal point.

Advantageous Effects of Invention

According to the present invention, correlative noise can be decreased by synthesizing a signal which is subjected to phasing processing for two or more steering directions including two directions other than a direction of a reception focal point direction. Therefore, it is possible to realize compensation of deterioration in the image quality of an ultrasound image caused by correlative noise resulting from wave-front distortion and improvement of an S/N ratio.

Brief Description of Drawings

[Fig. 1] Fig. 1 is a block diagram illustrating a configuration of a reception beamformer in a first embodiment.

[Fig. 2] Fig. 2 is an explanatory diagram illustrating a steering direction in the first embodiment.

[Fig. 3] Fig. 3 is an explanatory diagram illustrating a decreasing operation of correlative noise in the first embodiment.

[Fig. 4] Fig. 4(a) a perspective view illustrating a schematic configuration of an ultrasound imaging apparatus in the first embodiment, and Fig. 4(b) is a block diagram.

[Fig. 5] Fig. 5 is a block diagram illustrating a configuration of a reception beamformer in a second embodiment.

[Fig. 6] Fig. 6 is a flow chart illustrating an operation of the reception beamformer in Fig. 5.

[Fig. 7] Fig. 7 is a block diagram illustrating a configuration of a reception beamformer in a third embodiment.

[Fig. 8] Fig. 8 is a block diagram illustrating a configuration of a reception beamformer in a fourth embodiment.

[Fig. 9] Fig. 9 is a flow chart illustrating an operation of the reception beamformer in Fig. 8.

[Fig. 10] Fig. 10 is a block diagram illustrating a configuration of a reception beamformer in a fifth embodiment.

[Fig. 11] Fig. 11 is a block diagram illustrating a detailed configuration of the reception beamformer in Fig. 10.

[Fig. 12] Fig. 12 is a block diagram illustrating a configuration of a reception beamformer in a sixth embodiment.

[Fig. 13] Fig. 13 is a block diagram illustrating a detailed configuration of a phasing processing unit 204 in Fig. 12.

[Fig. 14] Fig. 14 is a block diagram illustrating a configuration of a reception beamformer in a seventh embodiment.

[Fig. 15] Fig. 15 is a block diagram illustrating a configuration of a reception beamformer in an eighth embodiment.

[Fig. 16] Fig. 16 is a block diagram illustrating a portion of a configuration of a reception beamformer in a ninth embodiment.

[Fig. 17] Fig. 17 is a perspective view of a console of an ultrasound imaging apparatus in the present embodiment.

[Fig. 18] Fig. 18 is a perspective view of the console and an image display unit in another specification example of the ultrasound imaging apparatus in the present embodiment.

[Fig. 19] Fig. 19 is a diagram (two-dimensional plotting) illustrating a detection result of a null angle obtained through delay adding processing of the present invention.

[Fig. 20] Fig. 20 is a diagram (a line profile) illustrating a detection result of the null angle obtained through the delay adding processing of the present invention.

[Fig. 21] Fig. 21 is a diagram (two-dimensional plotting) illustrating a detection result of the null angle obtained through an adaptive beamformer of the present invention.

[Fig. 22] Fig. 22 is a diagram (a line profile) illustrating a detection result of the null angle obtained through the adaptive beamformer of the present invention.

[Fig. 23] Figs. 23(a) and 23(b) are diagrams illustrating image contrast and null positions which are acquired from different test objects of the present invention.

Description of Embodiments

An ultrasound diagnostic apparatus in an embodiment of the present invention will be described.

(First Embodiment)

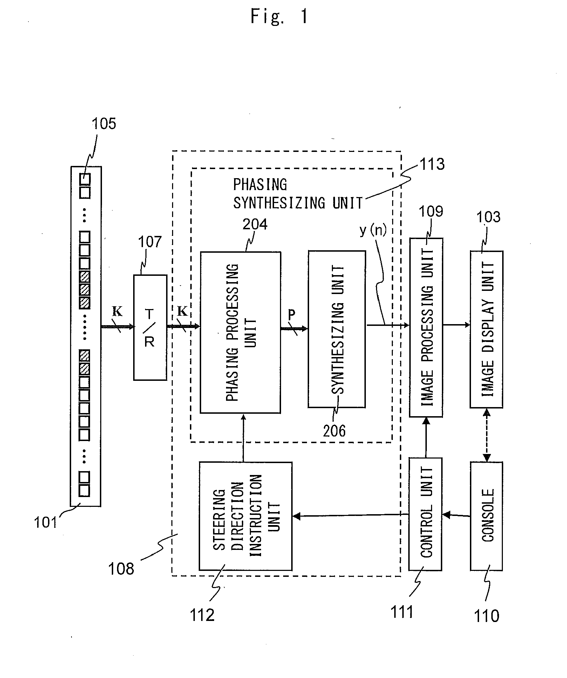

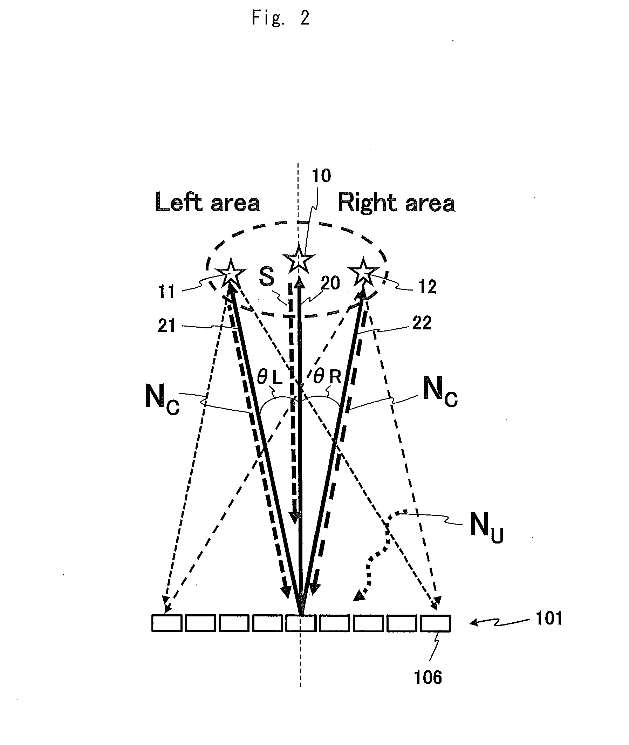

The ultrasound diagnostic apparatus in a first embodiment will be described with reference to Figs. 1 to 3. As shown in Fig. 1, the ultrasound diagnostic apparatus in the first embodiment is configured to include an ultrasound element array 101 in which multiple ultrasound elements (ultrasound transducers) 105 are arrayed along a predetermined direction, a reception beamformer 108 which performs phasing of a signal received by the ultrasound element array 101, and an image processing unit 109 which uses a phasing output that is output by the reception beamformer 108 and generates image data. The reception beamformer 108 includes a phasing synthesizing unit 113 and a steering direction instruction unit 112. The phasing synthesizing unit 113 performs synthesizing after performing phasing processing of the signal received by the ultrasound element array 101 for each of two or more steering directions. The steering direction instruction unit 112 instructs the phasing synthesizing unit 113 to perform phasing in the two or more steering directions. The two or more steering directions instructed by the steering direction instruction unit 112 include at least two directions (in Fig. 2, two directions each of which forms predetermined angles $\theta L$ and $\theta R$ on the right and left with respect to a direction 20 of a reception focal point 10 along an ultrasound element array 106 in an array direction) 21 and 22 other than the direction 20 of the reception focal point 10, as illustrated in Fig. 2. The aforementioned steering direction denotes a direction toward an imaging target (a test object) focusing on a predetermined ultrasound element 105. The steering direction is a direction within a surface including the array direction (that is, a longitudinal direction) of the ultrasound element 105 of the ultrasound element array 101 and a normal line of an ultrasound wave transmission/reception surface of the ultrasound element 105.

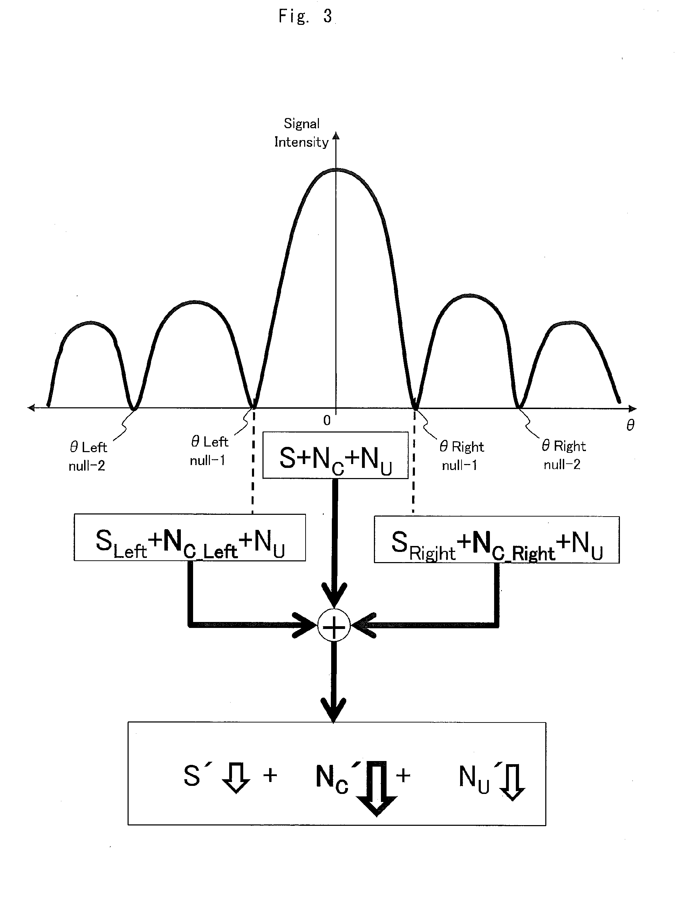

As long as each of the two directions 21 and 22 is a direction other than the direction 20 of the reception focal point 10, it is possible to acquire a certain effect of compensating deterioration in the image quality resulting from heterogeneity of a test object medium. In addition, as illustrated in Fig. 3, when each of the two directions 21 and 22 is a direction of a null angle θnull of a directivity profile of the ultrasound element array 101, the effect of compensating deterioration in the image quality becomes significant, thereby being more desirable. The aforementioned null angle θnull denotes an angle at which a reception signal indicates zero or a minimal value. In addition, the two or more steering directions may include the direction of the reception focal point 10 or need not include the direction thereof.

Hereinafter, an ultrasound imaging apparatus of the first embodiment will be described more specifically.

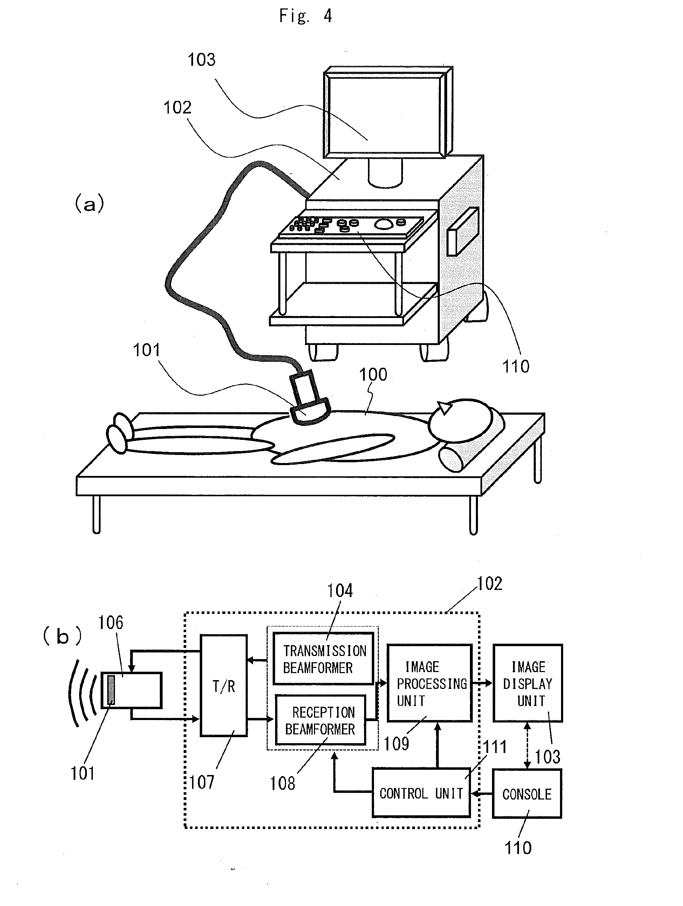

More descriptions will be given regarding the overall configuration of the ultrasound imaging apparatus with reference to Figs. 4 (a) and 4(b). Fig. 4 (a) a perspective view of the apparatus, and Fig. 4 (b) is a block diagram illustrating a schematic configuration of the inside thereof.

As shown in Fig. 4(a), the ultrasound imaging apparatus includes an ultrasound probe 106, an apparatus main body 102, an image display unit 103, and a console 110. Inside the apparatus main body 102, as shown in Fig. 1(b), a transmission beamformer 104, a transmission/reception separation circuit (T/R) 107, the reception beamformer 108, the image processing unit 109, and a control unit 111 for controlling operations thereof are arranged. The reception beamformer 108 and the image processing unit 109 are configured to be as described above in Fig. 1. The ultrasound probe 106 includes the ultrasound element array 101 illustrated in Fig. 1.

The transmission beamformer 104 generates a signal for a transmission beam. The transmission beam signal is transferred to the ultrasound probe 106 via the transmission/reception separation circuit 107. The ultrasound probe 106 transmits an ultrasound wave from the ultrasound element 105 of the ultrasound element array 101 toward the inside of a body of a test object 100. An echo signal which is reflected inside the body is received by the ultrasound element array 101 of the ultrasound probe 106. The reception signal passes through the transmission/reception separation circuit 107 again, thereby being subjected to phasing computation processing and the like in the reception beamformer 108.

As described above, the reception beamformer 108 includes the phasing synthesizing unit 113 and the steering direction instruction unit 112. The steering direction instruction unit 112 instructs the phasing synthesizing unit 113 to perform phasing in the two or more steering directions. As shown in Fig. 2, the two or more steering directions includes at least the two directions 21 and 22 each of which forms predetermined angles θL and θR greater than 0° with respect to the direction 20 of the reception focal point 10 along the ultrasound element array 106 of the reception focal point 10 in the array direction. The direction 20 of the reception focal point 10 may also be included. It is more desirable when predetermined angles θL and θR are in directions of the null angles θnull on the right and left of the ultrasound element array in Fig. 3. Here, for convenience, descriptions are given regarding an example in which the two steering directions are respectively set to be the directions on the right and left with respect to the direction of the reception focal point 10. However, the two or more steering directions may be set to be any directions with respect to the direction of the reception focal point 10. Particularly, when the ultrasound transducer is in a two-dimensional array, since there is no concept of right and left, the directions may be set to be any two or more arbitrary directions other than the reception focal point.

The phasing synthesizing unit 113 includes a phasing processing unit 204 and a synthesizing unit 206. The phasing processing unit 204 performs adding after performing phasing of each of the reception signals output by the multiple ultrasound elements 105 of the ultrasound element array 101 with respect to a point 11 of a steering direction 21. Then, phasing-adding is also performed with respect to a point 12 of the steering direction 22 in a similar manner. When there is another steering direction, phasing-adding is performed in a similar manner. Phasing processing of the phasing processing unit 204 may be performed through any processing method. For example, delay adding processing or adaptive phasing processing can be adopted.

The synthesizing unit 206 receives a result of phasing-adding from the phasing processing unit 204 for each of the multiple steering directions 21 and 22, and the like. Then, the synthesizing unit 206 synthesizes the result thereof by performing adding processing and the like, thereby outputting a phasing signal y(n).

An output (the phasing signal y(n)) of the synthesizing unit 206 is transferred to the image processing unit 109, and various types of image processing such as diverse filtering and scan converting are executed, thereby generating an ultrasound image. The ultrasound image is transferred to the image display unit 103 and is displayed therein.

In this manner, according to the present invention, by synthesizing a signal which is subjected to phasing processing, with the two directions 21 and 22 forming predetermined angles θL and θR with respect to the direction 20 of the reception focal point 10 along the ultrasound element array 106 in the array direction, it is possible to acquire the phasing signal y(n) in which noise (correlative noise Nc) indicating a correlationship is decreased, with respect to a signal S from the reception focal point 10 (the point to be received, the reception focal point). Accordingly, the phasing signal y (n) having a significant S/N ratio can be acquired. Thus, the image quality of an ultrasound image can be improved.

Followings are additional descriptions. Since the test object medium is heterogenous, a transmitted ultrasound signal is reflected by the reception focal point 10, and since the reflected ultrasound signal scatters over points on the periphery of the focal point, target information at the reception focal point 10 also exists on the periphery of the reception focal point 10. In addition, wave-front distortion occurs due to non-uniformity of sound velocity and the like resulting from heterogeneity of the test object medium. The wave-front distortion causes a reflected wave from the medium on the periphery of the reception focal point 10 to be mixed into a reception sound wave. The reflected wave from the medium on the periphery thereof is a noise signal (correlative noise) which is correlated to a signal from the reception focal point 10. Therefore, there is a need to reproduce the information of the focal point scattered over the periphery of the focal point and to decrease unnecessary correlative noise from the medium on the periphery of the focal point.

Wave-front distortion occurs not only through a propagation process of a reflected wave but also occurs through a propagation process of a transmission sound wave from when the sound wave is transmitted until the sound wave reaches the reception focal point 10, thereby obviously causing an increase of the aforementioned unnecessary correlative noise.

Therefore, according to the present invention, synthesizing is performed for a signal that is subjected to phasing processing for the two directions 21 and 22 each of which forms predetermined angles θL and θR with respect to the direction 20 of the reception focal point 10 along the ultrasound element array 106 in the array direction. For example, according to the present invention as shown in Fig. 3, when the ultrasound element array 101 includes the directivity profile in an angle direction, a signal which is subjected to phasing processing for each of the directions of the null angles θnull existing on both sides is synthesized with a signal which is subjected to phasing processing for the direction of a central portion, that is, a main lobe (an ultrasound propagation direction, θ = 0°), as necessary. When S represents a signal indicating information of the reception focal point 10, Nc represents correlative noise, and Nu represents non-correlative noise (white noise), an output signal of the phasing processing unit 204 in a case where the steering direction is oriented toward the main lobe direction 20 is represented by (S + Nc + Nu). In this case, the S/N ratio of the signal is becomes SNR = S/(S + Nc + Nu). The correlative noise Nc is correlative noise of which the progressing direction is put into disorder due to biological heterogeneity. The correlative noise Nc reaches the ultrasound element array 101 from a direction different from that of the reception focal point 10 as shown in Fig. 2. In addition, the non-correlative noise Nu is thermal noise, electrical noise, or the like. The non-correlative noise Nu is a signal having no directionality so as not to be mixed into the reception signal of the ultrasound element array 101.

In addition, as shown in Fig. 2, an output signal of the phasing processing unit 204 in a case where the steering direction is oriented toward the direction 21 of a predetermined angle θL (for example, a null angle θ_left_null-1, the first angle on the left side) is represented by (S_left + Nc_left + Nu). In addition, an output of the phasing processing unit 204 in a case where the steering direction is oriented toward the direction of a predetermined angle θR (for example, a null angle θ_right_null-1, the first angle on the right side) is represented by (S_right + Nc_right + Nu). However, S_left and S_right are signals indicating the information of the reception focal point 10.

Here, since the angle θL and the angle θR are oriented in directions different from each other in a transverse direction, S_left and S_right, and Nc_left and Nc_right are not only different from each other in a positional phase and a temporal phase of a signal but also have components of directionality reverse to each other. In other words, S_left and S_right, and Nc_left and Nc_right are asymmetrical to each other in both time and space. S_left and S_right, and Nc_left and Nc_right are signals which cancel out each other when being added. Therefore, as (S_left + Nc_left + Nu) and (S_right + Nc_right + Nu) are added by the synthesizing unit 206 of the phasing synthesizing unit 113, the component of the correlative noise Nc can be cancelled. In addition, particularly, when the angles θL and θR are set to the null angle θnull, the signals of S_left and S_right included in the output signal of the phasing processing unit 204 occupy a small proportion in the overall signal. Therefore, when synthesizing is performed for a signal which is subjected to phasing processing for the two directions 21 and 22 on the right and left each of which forms predetermined angles θL and θR, an effect of decreasing the correlative noise Nc becomes greater than an effect of decreasing the signals S_left and S_right indicating the information of the reception focal point 10. Therefore, as a result, a proportion contributing to improvement of SNR can be increased.

Moreover, as illustrated in Figs. 2 and 3, in addition to the directions of null beams on the right and left, the main lobe direction 20 is set to be the steering direction, and an output of the phasing processing unit 204 is synthesized for each of the steering directions. Therefore, correlative noise Nc' (= Nc_left + Nc_right + N) can be remarkably decreased. In addition, a signal S' (= S + S_left + S_right) indicating the information of the reception focal point 10 is not remarkably decreased as much as the correlative noise Nc' compared to S in the main lobe direction 20, and thus, an effect of improving SNR is increased further.

Meanwhile, the non-correlative noise Nu has no directionality and becomes ($1/\sqrt{n}$) times by being added n times. Accordingly, the non-correlative noise Nu is uniformly decreased by adding the outputs of the phasing processing unit 204 in the multiple steering directions.

As described above, compared to the original output signal (S + Nc + Nu) of the phasing processing unit 204 in the main lobe direction 20, the signal (S' + Nc' + Nu') = (1/3) x { (S + N + R) + (S_left + Nc_left + Nu) + (S_right + Nc_right + Nu)} which is obtained by synthesizing the output of the phasing processing unit 204 in the main lobe direction 20 in addition to the directions of null beams 21 and 22 on the right and left increases in SNR (Expression (1)). Here, the outputs of the phasing processing unit 204 for three directions are added while being 1/3 each in weight. However apodization can be performed in weights different from one another.

[Expression 1]

$$\frac{S}{S+N_C+N_U} \leq \frac{S'}{S'+N_C{}'+N_U{}'} \quad \cdots (1)$$

A phasing processing method for the phasing synthesizing unit 113 may be any method. However, by adopting adaptive phasing processing, the component of the signal S indicating the information of the reception focal point 10 can have increased directivity. In other words, while the desired signal component S in the synthesis result (1/3) x (S + S_left + S_right) of the desired signal component is negligibly decreased, an effect of decreasing the correlative noise Nc can be enhanced further, thereby being preferable.

In this manner, according to the first embodiment, by synthesizing the phasing processing results of the steering directions for at least two directions other than the direction of the reception focal point, and utilizing asymmetrical postulate of correlative noise in time and space, it is possible to decrease correlative noise resulting from wave-front distortion. Moreover, since information of the periphery of the reception focal point 10 can be collected, information which is reflected by the focal point and scatters over the periphery can also be collected. Accordingly, even though the medium of the test object is heterogenous, deterioration in the image quality can be prevented. Thus, it is possible to acquire the ultrasound imaging apparatus which is unlikely to be affected by correlative noise due to wave-front distortion, has high robustness, and has a relatively small processing load.

(Second Embodiment)

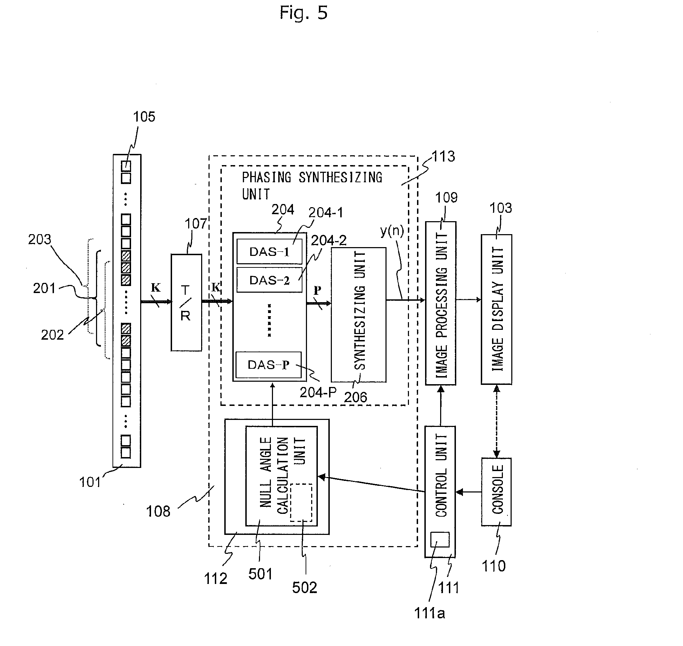

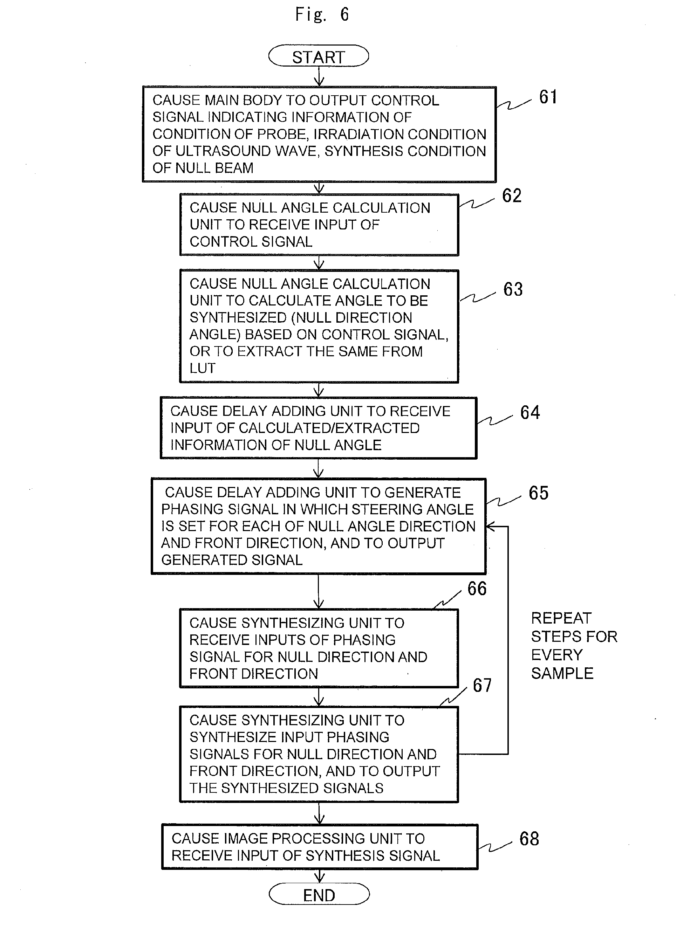

With reference to Figs. 5 and 6, the ultrasound imaging apparatus in a second embodiment will be described. Fig. 5 is a block diagram illustrating a configuration of the reception beamformer 108, and Fig. 6 is a flow chart illustrating an operation thereof.

According to the second embodiment, the phasing processing unit 204 inside the phasing synthesizing unit 113 of the first embodiment includes delay adding units 204-1 to 204-P in the number P in order to perform phasing processing through delay adding processing. The number P is the maximum number of the steering directions which can be designated by the steering direction instruction unit 112. In addition, the steering direction instruction unit 112 includes a null angle calculation unit 501 which calculates the null angle based on a condition of the ultrasound element array 101 and an irradiation condition of an ultrasound wave, and the directions of the null angles obtained by performing calculation are designated to the phasing processing unit 204 as the steering direction. The directions of the null angles are set to include at least two angles other than the direction of the reception focal point 10. The delay adding units 204-1 to 204-P of the phasing processing unit 204 are subjected to phasing through delay adding for each of the designated steering directions.

In addition, according to the second embodiment, an active channel setting unit 111a inside the control unit 111 sets active channels 201, 202, 203, and the like at a portion of the finite caliber of the ultrasound element array 101. The reception beamformer 108 performs reception beamforming for a reception signal of the active channel. In other words, elements 105 in the number K within a predetermined range among the ultrasound elements 105 configuring the ultrasound element array 101 which has received a reception echo with respect to one ultrasound transmission beam is set to be the active channel 201, and the reception beamformer 108 generates one item of image data (a raster: a phasing output y(n)) in the ultrasound propagation direction by using the reception signal of the active channel 201. As shown in Fig. 5, the active channel 202, the active channel 201, and the active channel 203 are sequentially configured while being gradually shifted in position on the ultrasound element array 101. The raster is generated for each of the active channels 202, 201, and 203, and the result in array becomes an ultrasound image.

The null angle calculation unit 501 calculates the null angle for the set active channel 201 and the like.

Hereinafter, descriptions will be given more specifically with reference to the flow in Fig. 6.

First, the control unit 111 generates a control signal which includes information indicating a condition of the probe, an irradiation condition of an ultrasound wave, and a selection condition of the null angle corresponding to the current imaging condition, thereby outputting the control signal to the null angle calculation unit 501 (Step 61).

The null angle calculation unit 501 receives an output of the control unit 111 (Step 62), and the null angle is calculated by using the following Expression (3) and the like while having the information included in the control signal as a parameter (Step 63). Expression (3) calculates the null angle only for transmission. However, the null angle can be calculated for reception as well by performing calculation in a similar manner. In addition, it is possible to calculate the superimposed transmission/reception null angle.

In calculation of the null angle, the primary null angle can be confirmed based on a frequency of an ultrasound wave, an imaging condition such as a frequency waveform, the number of the elements (the number of the active channel elements) of the ultrasound element 105 included in the currently used active channels 201 to 203, the caliber for transmission and reception, the channel intervals (the intervals in the ultrasound element 105), and characteristics of a frequency. In other words, the null angle can be calculated by using the control signal transmitted from the control unit 111.

For example, when the ultrasound element 105 is a square piston-type ultrasound element, directivity of an ultrasound beam transceived by the active channel 201 of the ultrasound element array 101 can be calculated through the following Expression (2). $D(\theta)$ is intensity of an ultrasound beam at a steering angle $\theta$.
[Expression 2]

$$D(\theta) = \frac{\sin(ka\sin\theta)}{(ka\sin\theta)} \quad \cdots \quad (2)$$

In Expression (2), $\theta$ represents the steering angle, a represents the caliber of the active channels (201 to 203), and k represents the number of waves. The value of k is obtained by dividing a sound velocity c by a frequency f of an ultrasound wave, thereby being represented by k = c/f.

The angle $\theta$null having a value of zero in Expression (2) is the null angle in this case. The null angle can be analytically obtained through Expression (3).
[Expression 3]

$$\theta_{null} = \arcsin\left(\frac{n\pi}{ka}\right) \quad \cdots \quad (3)$$

$$n = \pm 1, \ \pm 2, \ \pm 3 \cdots$$

As seen in Expression (3), there are multiple null angles $\theta$null instead of being one. As shown in Fig. 3, the null angles of the number n have characteristics of being present bisymmetrically on each of the right and left sides while having $\theta = 0°$ (ultrasound wave irradiation direction = direction of transmission focal point) as the center. Therefore, a condition for selecting the null angle is also input from the control unit 111 to the null angle calculation unit 501 as a control signal. The selection condition of the null angle is a condition for selecting the null angle which is used in processing by the phasing synthesizing unit 113, among the null angles $\theta$null-1, $\theta$null-2 to $\theta$null-n which are present on each of the right and left sides. For example, the condition is that instructions are given for selection such as using two null angles of n = + 1 and n = -1, or utilizing four null angles of n = $\pm$1 and $\pm$2. When the reference sign n is positive, it denotes that $\theta > 0°$, that is, the null angle is positioned on the right side from $\theta = 0°$. When the reference sign n is negative, it denotes that $\theta < 0°$, that is, the null angle is positioned on the left side from $\theta = 0°$.

The effect is maximized when n =$\pm$1. Therefore, it may be configured that only a processing unit of n = $\pm$1 included and two null angles of n = $\pm$1 must be used without performing null selection.

The null angle calculation unit 501 outputs the directions of the null angles which are selected from the null angles obtained by performing calculation in accordance with the selection condition of the null angle, with respect to the phasing processing unit 204 as the steering directions. As a form of the information designating the steering direction, for example, the information may be physical angle information such as $\theta = 10°$ and -10°. In addition, a control signal for activating (turn ON) the delay adding units 204-1 to 204-P of the phasing processing unit 204 and a signal which shares the aforementioned angle information may be directly transferred to the delay adding units 204-1 to 204-n. The aforementioned method exhibits a practically preferable embodiment. For example, $\theta$L = 10° is designated for the delay adding unit 204-1, and delay adding processing is instructed. Then, $\theta$R = -10° is designated for the delay adding unit 204-n, and delay adding processing is instructed.

The delay adding units 204-1 to 204-P of the phasing processing unit 204 generates a phasing signal in a case where the directions of the null angles designated from the null angle calculation unit 501 is set to be the steering direction, thereby outputting the generated signal (Step 65). In this case, it is desirable that a phasing signal is generated in the front direction ($\theta = 0°$: the direction of the reception focal point 20) as well. In other words, when the null angles are $\theta = 10°$ and -10°, in the delay adding units 204-1, 204-2, and 204-3, delay adding processing is performed for three directions, that is, the front directions of $\theta = 0°$, $\theta = 10°$, and $\theta = -10°$, and the results thereof are output to the synthesizing unit 206.

The synthesizing unit 206 receives inputs of the phasing signals for the null direction and the front direction from the delay adding units 204-1 to 204-P (Step 66). The synthesizing unit 206 performs synthesizing by adding the inputs, thereby outputting the added result (Step 67). When performing adding, for example, simple adding may be performed for each of three directions, that is, the front directions of $\theta = 0°$, $\theta = 10°$, and $\theta = -10°$ (the adding result is preferably multiplied by 1/3). Otherwise, for each steering direction, adding may be performed after multiplying the phasing signal by a predetermined weight. For example, it is possible to increase the proportion of the phasing signal for the front direction. In contrast, the synthesis result can be adjusted by multiplying the phasing signal for the null direction by a significant weight.

The synthesis signal computed by the synthesizing unit 206 is output to the image processing unit 109. The image processing unit 109 receives the synthesis signal (Step 68), and performs processing similar to that for the phasing result in the related art, thereby generating one item of image data (a raster) . Then, while being gradually shifted in position, each of the active channels 201, 202, and 203 acquires the raster. The rasters are arrayed so as to be processed into an ultrasound image. The ultrasound image is displayed by the image display unit 103. In this manner, the flow of the second embodiment according to the present invention is completed.

In the above-described flow in Fig. 6, when there is no variation in the information of the control signal of the control unit 111 with respect to the null angle calculation unit 501, that is, as long as no change is made in a condition of the probe, an irradiation condition of an ultrasound wave, and a selection condition of the null angle, the operation for the steps (Steps 61 to 65) until the null angle information is output to the delay adding units 204-1 to 204-P is performed in the same manner. Accordingly, as shown in Fig. 6, in Step 67, the reception beamformer 108 returns to Step 65 if the synthesizing unit 206 outputs a phasing signal to the image processing unit 109 for the reception signals in one sample (a set of the reception signals for one sampling), thereby repeating the operation for performing processing of the reception signals of the succeeding sample for each sample. Practically, in Step 65, processing of the reception signals for the succeeding sample may start before the outputting of the synthesis signal of the previous one sample is completed in Step 67. In other words, as described above, since the process of the flow in Fig. 6 proceeds over a number of stages, even though processing for the succeeding sample starts at a point of time in which processing at a certain sample time proceeds to the succeeding process, there is no inconvenience at all in terms of configuration and practicality.

In addition, in the above described configuration, calculation is performed for the null angle every time a change is made for the condition indicated by the control signal from the control unit 111. However, without being limited to the configuration, it is possible to arrange a memory 502 in the null angle calculation unit 501, and to store a table (the null angle LUT (LUT: Look Up Table) ) in which the calculation results of the null angles in a set correspond to conditions (a condition of the probe, an irradiation condition of an ultrasound wave, and a selection condition of the null angle) indicated by the control signal, in advance. In this manner, the null angle calculation unit 501 can read out the null angles corresponding to the conditions indicated by the control signal from the null angle LUT so as to set the null angles for the delay adding units 204-1 to 204-P. Therefore, the computation amount can be decreased.

Regarding the null angle LUT, the values prepared by obtaining the null angles in advance for all the conditions that can be set (a condition of the probe, an irradiation condition of an ultrasound wave, and a selection condition of the null angle) can be stored in the memory 502. However, without being limited thereto, the null angle LUT for only a portion of the conditions may be prepared and stored in the memory 502. In a case of a condition which is not included in the null angle LUT, the null angle calculation unit 501 may be configured to obtain the null angle by performing calculation.

In addition, it is also possible to have a configuration in which the null angle obtained by the null angle calculation unit 501 by performing calculation is caused to correspond to the condition indicated by the control signal at the moment and the result thereof is stored in the memory 502, thereby successively generating the null angle LUT.

As illustrated in Fig. 5, the delay adding units 204-1 to 204-P in the number P are prepared in the phasing processing unit 204. In this case, it is possible to allocate a set of the delay time corresponding to the steering angle in advance for each of the delay adding units 204-1 to 204-P in the number P. For example, in the delay adding unit 204-P at pth position, the maximum delay time for the steering angle is allocated. Accordingly, by only selecting the delay adding unit corresponding to the obtained steering angle from the delay adding units 204-1 to 204-P and instructing the delay adding unit regarding a computation operation, phasing processing can be instructed by the null angle calculation unit 501 regarding the steering direction. Without being limited to the aforementioned method, the delay adding units 204-1 to 204-P in the number P may have a configuration in which the sets of the delay times are calculated every time based on the steering direction designated by the null angle calculation unit 501 (on-the-fly computation).

As described above, in the second embodiment, by acquiring the phasing results to be obtained through delay adding processing of the steering directions for at least the two directions other than the direction of the reception focal point, synthesizing the acquired results, and utilizing asymmetrical postulate of correlative noise in time and space, it is possible to decrease correlative noise resulting from wave-front distortion. Moreover, information which is reflected by the focal point and scatters over the periphery can also be collected. Accordingly, even though the medium of the test object is heterogenous, deterioration in the image quality can be prevented. Thus, it is possible to acquire the ultrasound imaging apparatus which is unlikely to be affected by correlative noise due to wave-front distortion, has high robustness, and has a relatively small processing load.

Other configurations of the ultrasound imaging apparatus in the second embodiment are similar to those in the first embodiment, thereby omitting the descriptions.

(Third Embodiment)

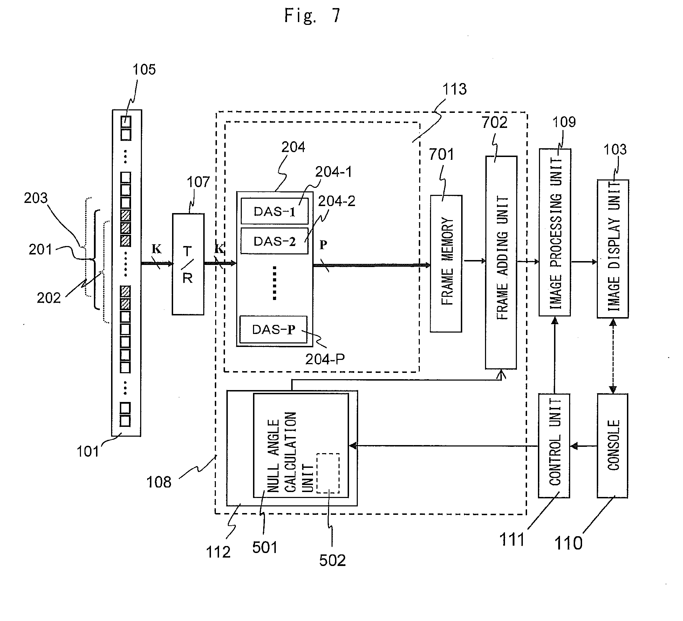

As a third embodiment, the ultrasound imaging apparatus will be described with reference to Fig. 7.

As shown in Fig. 7, in the third embodiment, the reception beamformer 108 includes a frame memory 701 and a frame adding unit 702 and performs aperture synthesis processing. The phasing synthesizing unit 113 does not include the synthesizing unit 206. Other configurations and operations thereof are similar to those in the second embodiment.

The output signal of a delay adding unit 204 in the present invention is the same as multi-look (multi-directional) reception data in known aperture synthesis processing. Accordingly, by applying the present invention when performing synthesizing of multi-look reception data among multiple transmissions in the known aperture synthesis processing, phasing synthesis processing for the two or more steering directions of the present invention can be realized through aperture synthesis processing.

Specifically, items of data in the number P which are generated for each ultrasound wave transmission by the delay adding units 204-1 to 204-P are successively saved in the frame memory unit 701 in Fig. 7. Subsequently, in the frame adding unit 702, the data saved for each transmission is subjected to adding processing for each angle at which the same point is viewed among the multiple transmissions, thereby performing aperture synthesis processing. By performing such aperture synthesis processing, reception data acquired from multiple transmission directions can be superimposed with respect to a certain imaging point, and thus, it is possible to acquire an ultrasound image having high resolution, a high SN ratio, and a high frame rate.

The data subjected to adding processing by the frame adding unit 702 is data similar to what is synthesized by the synthesizing unit 206 in the second embodiment (the data after being subjected to delay adding in multiple directions). In other words, the two or more steering directions from the steering direction instruction unit 112 are input to the frame adding unit 702 as synthesis angle information. The frame adding unit 702 reads out data regarding the designated two or more steering directions from the frame memory 701, thereby performing adding processing. Accordingly, aperture synthesis processing and data adding (adding of the two or more steering directions) in the null direction can be performed at the same time.

(Fourth Embodiment)

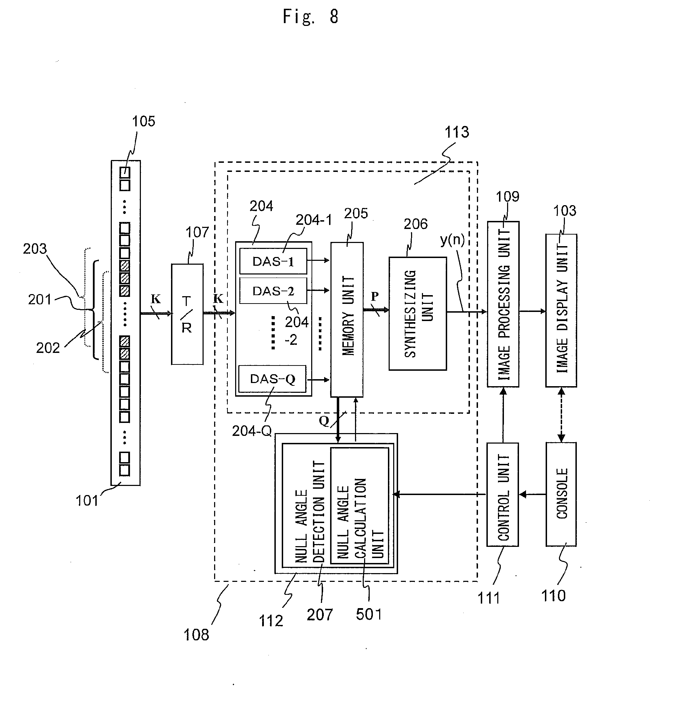

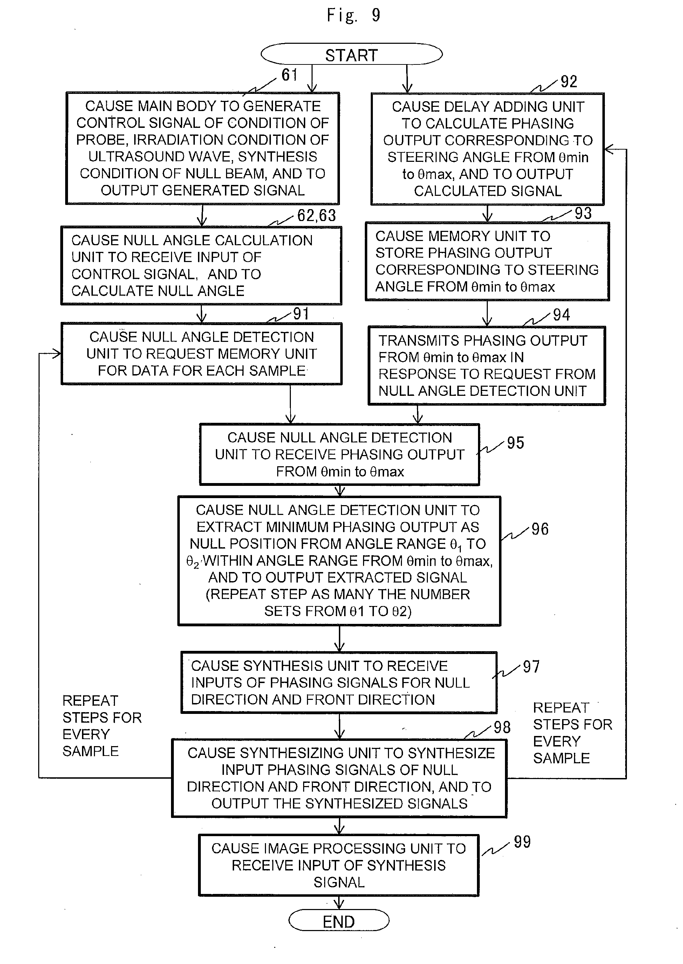

The ultrasound imaging apparatus in the fourth embodiment will be described with reference to Figs. 8 and 9.

In the fourth embodiment, the steering direction instruction unit 112 includes a null angle detection unit 207 and detects the null angle by using the phasing signal of the actual reception signal of the ultrasound element array 101. In order to enable the detection, the phasing synthesizing unit 113 includes the delay adding units 204-1 to 204-Q in the number Q in the phasing processing unit 204, and a memory unit 205 which stores the phasing outputs of the delay adding units 204-1 to 204-Q in the number Q.

Moreover, the null angle detection unit 207 includes the null angle calculation unit 501 described in the second embodiment and is configured to perform scanning of the null angle on the periphery of the null angle obtained by performing calculation so as to detect the null angle.

Other configurations are similar to those in the second embodiment, thereby omitting the descriptions.

Hereinafter, an operation of the reception beamformer 108 in the fourth embodiment will be described with reference to the flow chart in Fig. 9. In the flow of Fig. 9, the same reference numerals are applied to the steps similar to those in the flow in Fig. 6.

The second embodiment is configured to obtain the null angle from a condition of the probe, an irradiation condition of an ultrasound wave, and the like by performing calculation. Therefore, the null angle is decided depending on the condition of the apparatus side such as setting of the apparatus or an irradiation condition of an ultrasound wave. However, directivity of the actual reception signal is affected by heterogenous sound wave propagation in a living body so that directivity of a reception beam varies for each sample of the reception signal. Therefore, in the fourth embodiment, the null angle detection unit 207 detects the null angle for each sample of the reception signal based on the actual reception signal.

The control unit 111 generates and outputs control signals which indicate a condition of the probe, an irradiation condition of an ultrasound wave, and a selection condition of the null angle in accordance with the imaging condition at the moment, similarly to the second embodiment (Step 61). The null angle calculation unit 501 receives the control signals and obtains the null angle by performing calculation (Steps 62 and 63). The details for Steps 61 to 63 are the same as those described in the second embodiment. As the null angle calculation unit 501 obtains the null angle by performing calculation, the null angle detection unit 207 requests the memory unit 205 for data of the phasing signal.

Meanwhile, the delay adding units 204-1 to 204-Q of the phasing synthesizing unit perform delay adding processing for each of the steering directions in the number Q which are different from one another by a predetermined angle, within the preset angle range from $\theta$min to $\theta$max, thereby outputting the phasing signals (Step 92). The memory unit 205 stores the phasing signal for each steering angle within the angle range from $\theta$min to $\theta$max (Step 93). Physically,

for example, the angle range from θmin to θmax is the steering angle from -60° to 60°.

In accordance with the request from the null angle detection unit 207 in Step 91, the memory unit 205 outputs the phasing signal for the steering directions in the number Q within the angle range from θmin to θmax to the null angle detection unit 207 (Step 94).

The null angle detection unit 207 receives the phasing signal for the steering directions in the number Q within the angle range from θmin to θmax, the phasing signal within a predetermined angle range θ1 to θ2 is scanned out of the angle range from θmin to θmax, and extracts the minimum (minimal) phasing outputs as the null angle θnull_scan, thereby outputting the extracted results (Steps 95 and 96). This steps (θ1 and θ2) are repeated as many the number of the sets.

Here, the angle range from θ1 to θ2 is an angle range having the preset extent including the null angle which is obtained by the null angle calculation unit 501 through calculation. The angle range from θ1 to θ2 is the angle range which is assumed to have the actual null angle between the angles thereof. In other words, the set of (θ1 and θ2) is the angle range having the preset extent which becomes θ1 < θnull < θ2 with respect to θnull obtained by the null angle calculation unit 501. In addition, the number of sets of (θ1 and θ2) is the same number as the number of the null angles θnull which has decided depending on a null selection condition included in the control signal. Regarding this, it is the same as that described in the second embodiment.

By performing the processing, the null angle detection unit 207 detects the actual null angle θnull_scan in the same number as the number based on a selection condition of the null angle.

The null angle detection unit 207 instructs the memory unit 205 regarding the detected null angle θnull_scan. The memory unit 205 transmits the null angle information used in the delay adding unit 204. The transmission form of the detected null angle θnull_scan is the same as that in the second embodiment. For example, the null angle is transmitted as physical angle information such as θ = 10° and -10°.

The memory unit 205 selects the phasing signal for the steering directions of the null angle θnull_scan and the front direction (θ = 0°) which are transmitted from the null angle detection unit 207, thereby outputting the selected signal to the synthesizing unit 206. In other words, when the null angle is θ = 10° and -10°, the memory unit 205 outputs the phasing signals for three directions, that is, the front directions of θ = 0°, θ = 10°, and θ = -10°. In this case, the direction of θ = 0° is not necessarily included.

The synthesizing unit 206 receives the phasing signal of the steering direction of the null angle θnull_scan and the front direction (θ = 0°) from the memory unit 205 (Step 97) and performs synthesizing by performing adding, thereby outputting the result to the image processing unit 109 (Step 98). When performing adding, simple adding may be performed, or adding may be performed after multiplying each phasing signal by a predetermined weight.

The image processing unit 109 receives the phasing signal which is output by the synthesizing unit 206 and is synthesized. By using the received signal, similar to that in the second embodiment, an ultrasound image is generated, and the image is displayed by the image display unit 103 (Step 99). In this manner, the flow of processing in the fourth embodiment of the present invention is completed.

Regarding one sample of the reception signal, if outputting of the synthesis signal performed by the synthesizing unit 206 is completed in Step 98, the procedure returns to each of Steps 91 and 92, thereby performing processing for the reception signal of the succeeding sample.

As described above, in the fourth embodiment, since the null angle having the directivity of the actual reception signal can be detected, it is possible to set the null angle in accordance with variation of the null angle caused by an influence of heterogenous sound wave propagation in a living body. Accordingly, even though the medium of the test object is heterogenous, deterioration in the image quality can be prevented, and it is unlikely to be affected by correlative noise resulting from wave-front distortion. Thus, it is possible to acquire the ultrasound imaging apparatus having high robustness.

In the present embodiment, the null angle calculation unit may obtain the null angle by performing calculation, and the null angle detection unit 207 may scan the null angle only for a predetermined angle range θ1 to θ2 including the null angle obtained by performing calculation. Accordingly, a computation amount of the null angle detection unit 207 can be decreased. However, the present invention is not limited to the aforementioned configuration. The null angle detection unit 207 need not include the null angle calculation unit 501 and may scan the null angle for the entire angle range.

In addition, the null angle calculation unit 501 may be configured to include the memory 502 as described in the second embodiment and obtain the null angle with reference to the null angle LUT in the memory 502.

(Fifth Embodiment)

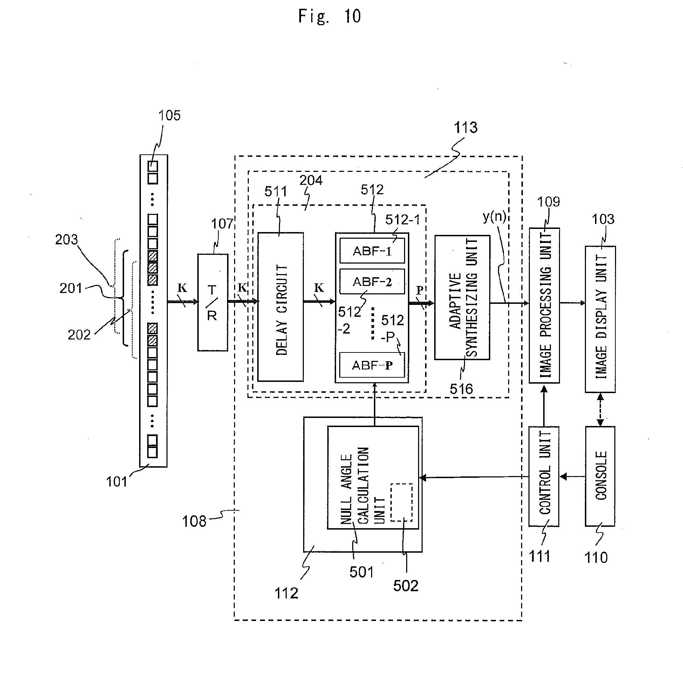

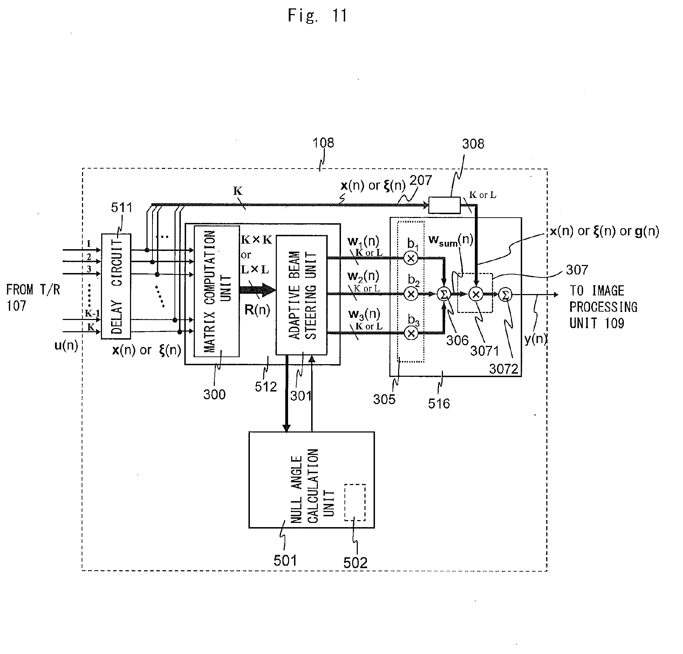

The ultrasound imaging apparatus in a fifth embodiment will be described with reference to Figs. 10 and 11.

The ultrasound imaging apparatus in the fifth embodiment is similar to that in the second embodiment. However, the point where the phasing processing unit 204 performs adaptive phasing processing is different from that in the second embodiment. In other words, as illustrated in Fig. 10, the phasing processing unit includes a delay circuit 511 and an adaptive phasing unit 512. The delay circuit 511 is a circuit which delays the reception signals in the number k such as the active channels 201 formed with the ultrasound elements 105 in the number k in accordance with the position of the reception focal point 10. Not only in the ultrasound wave transmission direction (0° direction), the reception focal point 10 may also be prepared in multiple numbers in the angle direction which is slightly tilted from the transmission direction (reception multiple beam processing). In this case, the phasing synthesizing units 113 are prepared in parallel manner for each tilted angle.

As shown in Fig. 11, the adaptive phasing unit 512 includes a matrix computation unit and an adaptive beam steering unit 301, and obtains an adaptive weight w for each of the steering directions designated by the null angle calculation unit 501. An adaptive synthesizing unit 516 uses the adaptive weight w so as to perform phasing for the reception signal output by the delay circuit 511 after performing delaying, and then, the adaptive synthesizing unit 516 performs synthesizing. An operation of the null angle calculation unit 501 is similar to that in the second embodiment.

In this manner, in the fifth embodiment, since the reception beamformer 108 performs adaptive phasing processing, it is possible to acquire an effect in which the correlative noise Nu can be more effectively decreased while the desired signal component S is negligibly decreased.

Hereinafter, adaptive phasing processing will be described specifically. When the number of the ultrasound elements 105 (the number of channels) configuring the active channel 201 is k, post-delaying reception data of the delay circuit 511 at a certain snapshot time n can be represented by a vector x(n) on the left side in the following Expression (4), by using an output $x_k(n)$ of the ultrasound element 105.

[Expression 4]

$$\mathbf{x}(n) = [x_1(n), x_2(n), ..., x_K(n)]^T \qquad \cdot\cdot\cdot\ (4)$$

The post-delaying reception data x(n) is input to each of the adaptive processing units 512-1 to 512-P. The adaptive processing units 512-1 to 512-P generate an adaptive weight vector $w_p(n) = [w_{p\_1}(n), w_{p\_2}(n)$ to $w_{p\_K}(n)]^T$ which is configured from a weight value wK (n) for each of the channels in the number k at the snapshot time n, for each of the steering directions designated by the null angle calculation unit 501. In other words, when the steering direction is in the number P, the vectors $w_1(n)$, $w_2(n)$ to $w_P(n)$ are generated.

After synthesizing the adaptive weight vectors $w_1(n)$, $w_2(n)$ to $w_P(n)$, the adaptive synthesizing unit 516 performs inner product computation (apodization computation) with the post-delaying reception data x(n), thereby acquiring the adaptive beamformer output y(n) at a certain snapshot time n.

As shown in Fig. 11, the adaptive processing units 512-1 to 512-P of the adaptive phasing unit 512 includes a matrix computation unit 300 and the adaptive beam steering unit 301. Each of the adaptive processing units 512-1 to 512-P may include the matrix computation unit 300 and the adaptive beam steering unit 301. Otherwise a set of the matrix computation unit 300 and the adaptive beam steering unit 301 may be shared by the adaptive processing units 512-1 to 512-P.

In the matrix computation unit 300, the post-delaying reception data vector x (n) obtains a spatial covariance matrix R(n) through the following Expression (5) . When obtaining the matrix R(n), the vector x(n) may be used as the actual signal as it is, or a vector which is converted into data in the complex number by executing Hilbert transformation, baseband modulation, or the like. Here, as a more general form, descriptions will be given by exemplifying a case where the vector x (n) is converted into a complex data $\xi(n)$ so as to obtain the spatial covariance matrix R(n) as shown in Expression (5) . The matrix R(n) in Expression (5) is obtained by taking an ensemble average of the product between the complex vector $\xi(n)$ which is represented by Expression (6) and the (complex) conjugate transposition vector $\xi^H(n)$ thereof.

[Expression 5]

$$\mathbf{R}(n) = E[\xi(n)\xi^H(n)] = E \left\{ \begin{pmatrix} \xi_1(n)\xi_1^*(n) & \xi_1(n)\xi_2^*(n) & \cdots & \xi_1(n)\xi_k^*(n) \\ \xi_2(n)\xi_1^*(n) & \xi_2(n)\xi_2^*(n) & \cdots & \xi_2(n)\xi_k^*(n) \\ \vdots & \vdots & \ddots & \vdots \\ \xi_K(n)\xi_1^*(n) & \xi_K(n)\xi_2^*(n) & \cdots & \xi_K(n)\xi_K^*(n) \end{pmatrix} \right\}$$

$$= \frac{1}{N} \sum_{s=-S}^{S} \xi(n+s)\, \xi^H(n+s) \qquad \cdot\cdot\cdot\ (5)$$

[Expression 6]

$$\xi(n) = \left[\xi_1(n), \xi_2(n), \ldots, \xi_K(n)\right]^T \qquad \cdots \quad (6)$$

In Expression (5), an ensemble average number N can be uniformly averaged as shown on the far right side in Expression (5), as the points in total N = 2S + 1 including the samples in the number S in front and rear of the vector $\xi(n)$ in the target snapshot. Moreover, as the method of averaging the temporal direction, in addition thereto, it is possible to adopt a method in which after each sample in the temporal direction is multiplied by an arbitrary weight such as a trapezoidal weight, adding average is obtained. The spatial covariance matrix R (n) output from the matrix computation unit 300 is successively input to the adaptive beam steering unit 301.

In the adaptive beam steering unit 301 which receives the spatial covariance matrix R(n), the weight vector $w_p(n)$ is calculated by using the MVDR method. A steering vector $a_p$ designated by the null angle calculation unit 501 is represented by Expression (7).

[Expression 7]

$$\mathbf{a}_p = \left[ \exp\left\{\psi_1\left(\theta_p, \phi_p, f_p\right)\right\}, \exp\left\{\psi_2\left(\theta_p, \phi_p, f_p\right)\right\}, \ldots, \exp\left\{\psi_K\left(\theta_p, \phi_p, f_p\right)\right\} \right]$$

$$= \left[ 1, \exp\left(-j\frac{2\pi}{\lambda_p}d\sin\theta_p\right), \exp\left(-j\frac{2\pi}{\lambda_p}\cdot 2d\sin\theta_p\right), \ldots \exp\left(-j\frac{2\pi}{\lambda_p}(K-1)d\sin\theta_p\right) \right]$$

$$\cdots \quad (7)$$

In Expression (7) shown above, the reference sign p represents the number of the steering vectors and is an integer satisfying 0 < p < P + 1 as the total number P. As shown in Expression (7), the steering vector $a_p$ is a directional vector which has the vector element (0 to (K - 1)) in the number K equal to the number of the active channels. The steering vector $a_p$ is represented by a function of a reception frequency $f_p$, and an angle (hereinafter, referred to as a steering angle) ($\theta_p$, $\phi_p$) formed by the normal line vector direction on the surface of the ultrasound element 105 and the steering vector.

The reference sign $\theta_p$ represents an open angle from the normal-line vector, and the reference sign $\theta_p$ represents a swiveling angle from the array direction of the ultrasound element 105. When the arrangement of the ultrasound elements 105 of the ultrasound probe 106 is a one-dimensional straight-line (linear) array, the steering angle is represented by the last expression in Expression (7). Here, the reference sign $\lambda_p$ represents a wavelength of a sound wave corresponding to the frequency $f_p$, and the reference sign d represents the interval (the pitch of the elements) between the element centers of the ultrasound element 105.

Regarding the direction of the above-described steering vector $a_p$, the weight vector $w_p$ (n) obtained by the MVDR method is calculated through Expression (8) herein. Accordingly, by computing the weight vector $w_p(n)$ for each of the steering vectors $a_p$ different from one another by the adaptive processing units 512-1 to 512-P, it is possible to acquire the adaptive weight vector $w_1(n)$ to $w_P(n)$ as many the number of P of the steering vector $a_p$.

[Expression 8]

$$\mathbf{w}_p(n) = \frac{\mathbf{R}^{-1}(n)}{\mathbf{a}_p^H \mathbf{R}^{-1}(n)\mathbf{a}_p} \qquad \cdots \quad (8)$$

In Expression (8), the reference sign R(n) represents a spatial covariance matrix at a certain snapshot n in the temporal direction generated through Expression (5), and the superscript -1 represents an inverse matrix.

The reception focal point 10 is on the central axis of the active channel 201, that is, when the number of the active channels is an even number, the reception focal point 10 is positioned on the normal line (the normal line orthogonal to the active channel surface) passing through the center point between the elements on the K/2nd and (K + 2)/2nd among the ultrasound elements 105 in the number K configuring the active channels 201. In addition, when the number of the active channels is an odd number, the reception focal point 10 is positioned on the normal line (the normal line orthogonal to the active channel surface) passing through the center of the ultrasound element 105 on (K + 1)/2nd among the ultrasound elements 105 in the number K configuring the active channels 201.

Fig. 11 illustrates a case of the total number P = 3 for the steering direction, illustrating an example of the three adaptive weight vectors $w_1(n)$ to $w_P(n)$. However, actually, the adaptive weight vectors in the number designated by the null angle calculation unit 501 are output from the adaptive beam steering unit 301.

Since delaying processing is executed in a delay circuit 204, for example, the reception data vector x(n) at a certain time n in linear scanning becomes data in which wave-fronts are aligned in a direction of $\theta = 0°$ that is a direction of the normal line vector. Accordingly, in Expression (7), when $\theta_p = 0°$, the steering vector $a_p$ is $a_p = [1, 1, \text{and so forth } 1]^T$, thereby obtaining the adaptive weight vector w(n) regarding the focal point direction. The adaptive weight vector w(n) regarding the focal point direction coincides with the adaptive weight vector w(n) obtained by the MVDR method in the related art.

The adaptive weight vectors $w_1(n)$ to $w_P(n)$ in the number P output from the adaptive beam steering unit 301 are input to the adaptive synthesizing unit 516. As shown in Fig. 11, the adaptive synthesizing unit 516 adds the adaptive weight vectors $w_1(n)$ to $w_P(n)$ in the number P by a weight synthesizing unit 306 and obtains an arithmetic average, thereby calculating a synthesis weight vector $w_{sum}(n)$ as shown in Expression (9). For example, as shown in Fig. 11, the synthesis weight vector $w_{sum}(n)$ of the adaptive weight vector having P = 3 becomes $w_{sum}(n) = \{w_1(n) + w_2(n) + w_3(n)\}/3$.

[Expression 9]

$$\mathbf{w}_{sum}(n) = \sum_{p=1}^{P} \mathbf{w}_p(n) = \frac{1}{P}\left\{\mathbf{w}_1(n) + \mathbf{w}_2(n) + \cdots \mathbf{w}_p(n) \cdots + \mathbf{w}_P(n)\right\} \quad \cdot \cdot \cdot \quad (9)$$

In the previous stage where the synthesis weight vector $w_{sum}(n)$ is obtained, a fixed apodization multiplying unit 305 may be arranged so as perform multiplication for the fixed apodization with respect to each of the weight vectors $w_p(n)$. For example, it is possible to perform multiplication for a fixed apodization $b_p$ applied with a distribution in which the value of the adaptive weight vector $w_P(n)$ having the direction of $\theta = 0°$ is increased and the values of other directions are decreased. This is realized by performing computation through Expression (10).

[Expression 10]

$$\mathbf{w}_{sum}(n) = \sum_{p=1}^{P} b_p \mathbf{w}_p(n) = \frac{1}{P}\left\{b_1\mathbf{w}_1(n) + b_2\mathbf{w}_2(n) + \cdots b_p\mathbf{w}_p(n) \cdots + b_P\mathbf{w}_P(n)\right\} \quad \cdot \cdot \cdot \quad (10)$$

For example, in the configuration in Fig. 11, the fixed apodization weights $b_1$, $b_2$, and $b_3$ are prepared in the fixed apodization multiplying unit 305. In this case, the synthesis weight output from the weight synthesizing unit becomes $w_{sum}(n) = \{b_1w_1(n) + b_2w_2(n) + b_3w_3(n)\}/3$.

Subsequently, the synthesis weight vector $w_{sum}(n)$ is input to an apodization computation unit 307 inside the adaptive synthesizing unit 516. The apodization computation unit 307 includes a multiplying unit 3071 and performs an inner product computation as shown in Expression (11) between the synthesis weight vector $w_{sum}(n)$ and the post-delaying reception data vector x(n) which is transmitted through a bypass line 207 from the delay circuit 204, thereby acquiring the phasing output y(n). Specifically, the multiplying unit 3071 calculates the product between the channel elements (1 to K) of each of the weight vectors and the post-delaying reception data vectors. An adding unit 3072 calculates the sum total of the products for the elements in the number k acquired by the multiplying unit 3071 of the apodization computation unit 307, as the final output (the phasing output y(n), a scalar value) of the adaptive synthesizing unit 516.

[Expression 11]

$$y(n) = \mathbf{w}_{sum}^{H}(n)\mathbf{x}(n) \quad \cdot \cdot \cdot \quad (11)$$

The phasing output y(n) of the apodization computation unit 307 is output to the image processing unit 109. The above-described processing is repeated from the first sample n = 1 to the last sample n = N among the N samples configuring one raster. Then, the apodization computation unit 307 outputs the sequential phasing outputs Y(1), Y(2) to Y(N) to the sequential image processing unit 109. The phasing output y (n) of one raster which can acquired through Expression (11) is acquired individually while shifting the active channel 201 to the active channels 202 and 203 on the ultrasound element array 101. In the image processing unit 109, all the rasters are arrayed by a scan converter adapted for the scanning method of the ultrasound probe 106, thereby generating a two-dimensional image. In addition, various types of back-end image processing such as diverse filtering and computation of measurement application are performed.

Eventually, the image display unit 103 displays an ultrasound image or a computation result of the measurement application.

In this manner, in the fifth embodiment, by using adaptive phasing processing, while the desired signal component S is prevented from decreasing, it is possible to acquire an effect in which the correlative noise Nu can be more effectively decreased.

In the fifth embodiment, as another technique for computing the algorithm in the above-described matrix computation unit 300, it is possible to perform spatial averaging computation by using a sub-array matrix. A sub-array matrix $R^{\wedge}_{SUBl}$ in spatial averaging computation is represented by the product of the subspace vector $\xi^{\wedge}_l(n)$ (Expression (13)) as shown in Expression (12). The subspace vector $\xi^{\wedge}_l(n)$ is a vector which is obtained by partially eliminating the component (corresponding to the element L) out of the post-delaying reception data (represented by the general complex signal vector $\xi(n)$ in this case, but the actual signal vector x(n) may be adopted in place thereof) with respect to the active channels in the number K. Accordingly, the total number of the subspace vector becomes the number K - L + 1 (0 < 1 (small letter l) < K - L + 1).

[Expression 12]

$$\mathbf{R}^{\wedge}_{\mathrm{SUB}l}(n) = \boldsymbol{\xi}^{\wedge}_{l}(n)\boldsymbol{\xi}^{\wedge H}_{l}(n) \qquad \cdots (12)$$

[Expression 13]

$$\boldsymbol{\xi}^{\wedge}_{l}(n) = [\xi_{l}(n), \xi_{l+1}(n), ..., \xi_{l+L-1}(n)]^{T} \qquad \cdots (13)$$

When the main diagonal component of the sub-array matrix is caused to coincide with the main diagonal component of the spatial covariance matrix R (n) so as to be shifted by one sample, it becomes spatial averaging processing for the sub-array matrix in the number K - L + 1, thereby acquiring the sub-array spatial covariance matrix $R^{\wedge}(n)$ in Expression (14). When computing the sub-array spatial covariance matrix $R^{\wedge}(n)$ by the adaptive beam steering unit 301, the sub-array spatial covariance matrix $R^{\wedge}(n)$ can replace R (n) in the above-described Expression (8), and thus, it is possible to compute the weight vector $w_p(n)$ as shown in Expression (15). In this case, the output of the matrix computation unit 300 has a size of L x L, and the number of elements configuring the weight vector $w_p(n)$ becomes L.

[Expression 14]

$$\mathbf{R}^{\wedge}(n) = \frac{1}{N(K-L+1)} \sum_{s=-S}^{S} \sum_{l=1}^{K-L+1} \mathbf{R}^{\wedge}_{\mathrm{SUB}l} \qquad \cdots (14)$$

$$= \frac{1}{N(K-L+1)} \sum_{s=-S}^{S} \sum_{l=1}^{K-L+1} \boldsymbol{\xi}^{\wedge}_{l}(n)\boldsymbol{\xi}^{\wedge H}_{l}(n)$$

[Expression 15]

$$\mathbf{w}_{p}(n) = \frac{\mathbf{R}^{\wedge-1}(n)}{\mathbf{a}^{H}_{p}\mathbf{R}^{\wedge-1}(n)\mathbf{a}_{p}} \qquad \cdots (15)$$

In addition, as another example of the spatial averaging method, a frontward-rearward spatial averaging method may be adopted. In this case, a rearward sub-array matrix R to $_{SUBl}$ (n) can be obtained by the product of a rearward the subspace vector $\xi$ to $_l(n)$ as shown in Expression (16). The rearward subspace vector is represented by Expression (17). In addition, as shown in Expression (18), by performing computation similarly to the frontward spatial averaging, the rearward sub-array spatial matrix R to (n) can be calculated as shown in Expression (18). Subsequently, as shown in Expression (19), by performing arithmetic averaging with the sub-array spatial matrix $R^{\wedge}(n)$ obtained in the above-described frontward spatial averaging, it is possible to eventually obtain a frontward/rearward sub-array spatial covariance matrix $R_{FB}$ (n) . Similar to frontward spatial averaging, by replacing R(n) in Expression (8) with the frontward/rearward sub-array spatial covariance matrix $R_{FB}(n)$ when computing the adaptive beam steering unit 301, the weight vector $w_p(n)$ can be computed as shown in Expression (20). In this case as well, the output of the matrix computation unit 300 has a size of L x L, and the number of elements configuring the weight vector $w_p(n)$ becomes L.

[Expression 16]

$$\mathbf{R}_{SUB\mathit{l}}^{\sim}(n) = \xi_{\mathit{l}}^{\sim}(n)\xi_{\mathit{l}}^{\sim H}(n) \qquad \cdots (16)$$

[Expression 17]

$$\xi_{\mathit{l}}^{\sim}(n) = [\xi_{K-\mathit{l}-1}(n), \xi_{K-\mathit{l}-2}(n), ..., \xi_{K-\mathit{l}-K}(n)]^{T} \qquad \cdots (17)$$

[Expression 18]

$$\mathbf{R}^{\sim}(n) = \frac{1}{N(K-L+1)} \sum_{s=-S}^{S} \sum_{\mathit{l}=1}^{K-L+1} \mathbf{R}_{SUB\mathit{l}}^{\sim}(n)$$

$$= \frac{1}{N(K-L+1)} \sum_{s=-S}^{S} \sum_{\mathit{l}=1}^{K-L+1} \xi_{\mathit{l}}^{\sim}(n)\xi_{\mathit{l}}^{\sim H}(n) \qquad \cdots (18)$$

[Expression 19]

$$\mathbf{R}_{FB}(n) = \frac{\mathbf{R}^{\wedge}(n) + \mathbf{R}^{\sim}(n)}{2} \qquad \cdots (19)$$

[Expression 20]

$$\mathbf{w}_p(n) = \frac{\mathbf{R}_{FB}^{-1}(n)}{\mathbf{a}_p^{H}\mathbf{R}_{FB}^{-1}(n)\mathbf{a}_p} \qquad \cdots (20)$$

The weight vector $w_p$ in multiple numbers (the number P) calculated through Expression (15) or Expression (20) by using the spatial averaging method is transmitted to the adaptive synthesizing unit 516, similarly to a case where the spatial averaging method is not used.

Here, when the spatial averaging method is used, the number of elements of the weight vector $w_p$ is L. Accordingly, in order to eventually perform computation in an inner product computation unit 307, a block is additionally required so as to perform computation (Expression (21)) for producing from $\xi$ (n) having the components in the number K to the vector g(n) having the components in the number L. For example, as illustrated in Fig. 11, a dimension condensing unit 308 can be configured to be arranged in the midst of the bypass line 207 while being in the previous stage of the apodization computation unit 307 so that the dimension condensing unit 308 generates from $\xi$ (n) having the elements in the number K to the vector g (n) having the elements in the number L. The dimension condensing unit 308 can be arranged inside the synthesizing unit 206.

The phasing output y (n) when the spatial averaging method is used can be represented by Expression (22).

[Expression 21]

$$\mathbf{g}(n) = \sum_{\mathit{l}=1}^{K-L+1} \xi_{\mathit{l}}^{\wedge}(n) \qquad \cdots (21)$$

[Expression 22]

$$y(n) = \mathbf{w}_{\text{sum}}^{H}(n)\mathbf{g}(n) \qquad \cdots \; (2\,2)$$

In this manner, in the matrix computation unit 300, by performing spatial averaging processing of the sub-array matrix, it is possible to acquire an effect of preventing correlative noise included in an ultrasound wave reception signal. Accordingly, by combining spatial averaging processing of the sub-array matrix in the configuration in which multiple adaptive weight vectors on the periphery of the focal point is synthesized, it is possible to acquire an ultrasound image which is negligibly affected by noise.

As described above, in the fifth embodiment, MVDR is exemplified as an example of a technique for adaptive beam forming. However, the algorithm applied to the adaptive beam steering unit 301 is acceptable as long as the algorithm used the spatial covariance matrix calculated by the matrix computation unit 300. Moreover, any types of other techniques such as the MMSE method; the APES method; the eigen space-MV method (ESMV, EIBMV) utilizing the spatial covariance matrix, the eigen value, and the eigen vector thereof; the ESPRIT method, and the MUSIC method, can be used.

(Sixth Embodiment)

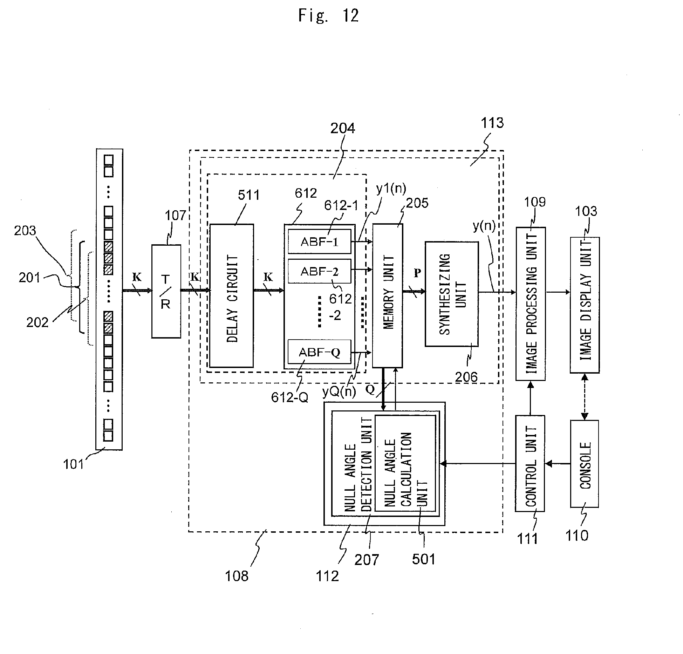

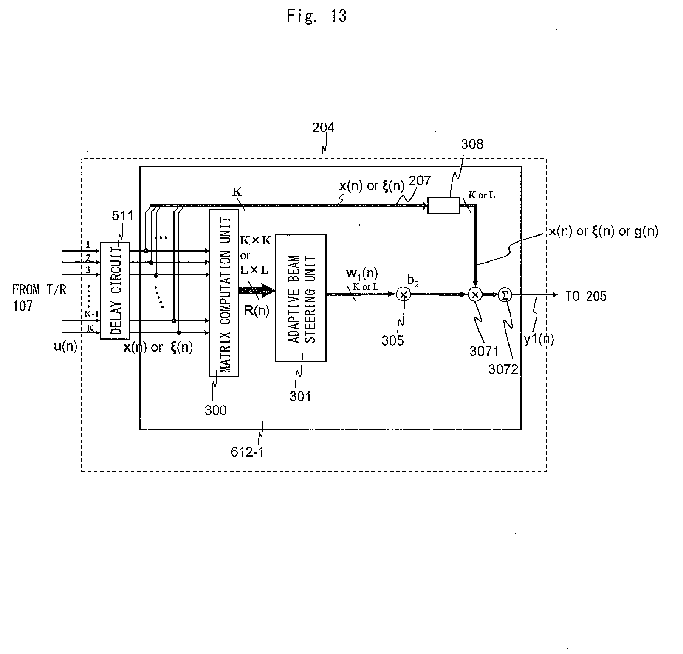

The ultrasound imaging apparatus in a sixth embodiment will be described with reference to Figs. 12 and 13. The ultrasound imaging apparatus in the sixth embodiment is configured to cause the null angle detection unit 207 to detect the actual null angle by using the phasing signal of the phasing processing unit 204, similarly to the fourth embodiment. However, the point where the phasing processing unit 204 performs adaptive phasing processing is different from that in the fourth embodiment.

Specifically, as illustrated in Fig. 12, the phasing processing unit 204 includes the delay circuit 511 and an adaptive phasing unit 612. The adaptive phasing unit 612 includes adaptive processing units 612-1 to 612-Q in the number Q. The delay circuit 511 is configured and operated similar to that in the fifth embodiment.

Similarly to the fourth embodiment, the adaptive processing units 612-1 to 612-Q obtain the phasing outputs for the steering directions in the number Q within the angle range from θmin to θmax. As illustrated in Fig. 13, each of the adaptive processing units 612-1 to 612-Q not only obtains the adaptive weight vectors w1(n) to wQ(n) by the matrix computation unit 300 and the adaptive beam steering unit 301 similar to the adaptive processing units 512-1 to 512-P in the fifth embodiment, but each of the adaptive processing units 612-1 to 612-Q also includes the multiplying unit 3071 and the adding unit 3072. The multiplying unit 3071 of the adaptive processing unit 612-1 performs inner product computation between the weight vector w1 (n) and the post-delaying reception data vectors x(n) which is transmitted from the delay circuit 511 through the bypass line 207. The adding unit 3072 performs adding of the vectors and outputs the result to the phasing output y1(n). Similarly, the adaptive processing units 612-2 to 612-Q outputs the phasing outputs y2 (n) to yQ(n). In this case, the fixed apodization multiplying unit 305 may be arranged so as to perform multiplication of the fixed apodization with respect to the weight vector w1(n).

Operations of the memory unit 205 and the null angle detection unit 207 are similar to those in the fourth embodiment, thereby omitting the descriptions.

In the sixth embodiment, since the phasing signals obtained through adaptive phasing processing for the steering direction are synthesized, it is possible to enhance the effect to decrease correlative noise resulting from wave-front distortion. In addition, since the null angle is detected by using the phasing signal obtained through adaptive phasing processing, there is an effect of achieving high detection accuracy of the null angle as well.

(Seventh Embodiment)

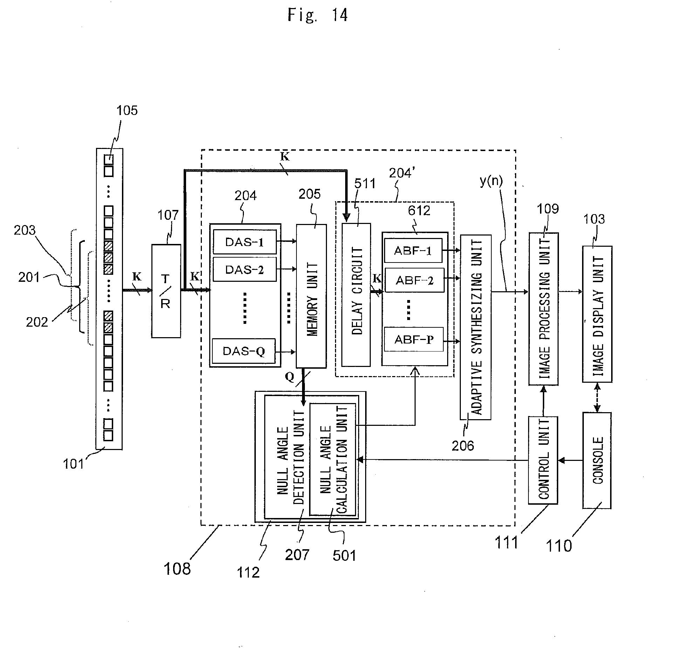

The ultrasound imaging apparatus in a seventh embodiment will be described with reference to Fig. 14. The seventh embodiment includes the reception beamformer 108 which is a combination of the configuration of the fourth embodiment in Fig. 4 and the configuration of the sixth embodiment in Fig. 12. The reception beamformer 108 includes the phasing processing unit 204 which acquires the phasing signal for the steering direction in the number Q through delay adding shown in Fig. 8, and the memory unit 205 which stores the phasing signal. The null angle detection unit 207 detects the null angle based on the phasing signal in the number Q subjected to phasing processing through delay adding. Meanwhile, the reception beamformer 108 includes a phasing processing unit 204' which acquires the phasing signal through adaptive processing in Fig. 12. The adaptive phasing unit 612 obtains the phasing signal y1(n) to yP(n) through adaptive phasing processing for each of the steering directions of the null angles in the number P designated by the null angle detection unit 207. The synthesizing unit 206 performs adding and synthesizing of the phasing signal y1(n) to yP(n), thereby obtaining the phasing signal y(n).

In the present embodiment, the null angle is detected by the phasing signal obtained through delay adding processing, and adaptive phasing processing is performed only for the steering direction designated by the null angle detection unit 207. Therefore, it is possible to synthesize the phasing signals obtained through adaptive phasing processing for the steering direction with a computation amount less than that in the sixth embodiment. Thus, it is possible to enhance the effect of decreasing correlative noise resulting from wave-front distortion with a small computation amount.

The adaptive phasing unit 612 and the synthesizing unit 206 in Fig. 14 can replace the adaptive phasing unit 512 and the adaptive synthesizing unit 516 in Fig. 10.

(Eighth Embodiment)

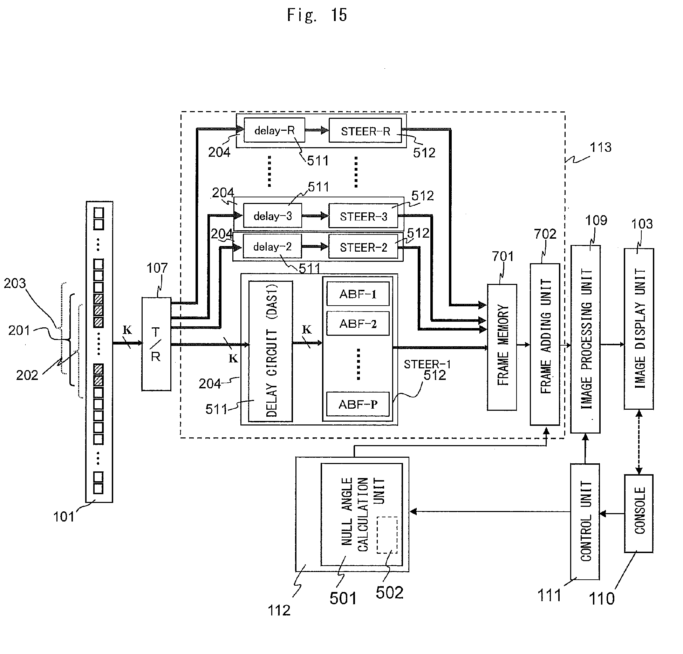

The ultrasound imaging apparatus in an eighth embodiment will be described with reference to Fig. 15.

The eighth embodiment is configured to perform aperture synthesizing in the reception beamformer 108 of the fifth embodiment in Fig. 10.

With reference to Fig. 15, the ultrasound imaging apparatus in the eighth embodiment of the present invention will be specifically described. Similar to the third embodiment, in the eighth embodiment, items of data in the number R which are generated for each ultrasound wave transmission are successively saved in the frame memory unit 701 in Fig. 15. In the frame adding unit 702, the data saved for each transmission is subjected to adding processing for each angle at which the same point is viewed among the multiple transmissions, thereby performing phasing synthesis processing and aperture synthesis processing for the two or more steering directions of the present invention at the same time. By performing aperture synthesis processing, reception data acquired from multiple transmission directions can be superimposed with respect to a certain imaging point, and thus, it is possible to acquire an ultrasound image having high resolution, a high SN ratio, and a high frame rate.

As shown in Fig. 15, the eighth embodiment has a configuration different from that of the third embodiment in Fig. 7 in phasing processing of the frame memory 701 performed in the previous stage. In the third embodiment, the multiple delay adding units 204-1 to 204-P are provided in the phasing synthesizing unit 113, but in the eighth embodiment, the phasing synthesizing unit 113 includes the phasing processing units 204 in the number R which includes the delay circuit 511 of the fifth embodiment in Fig. 5 and the adaptive phasing unit 512.

The frame memory unit 701 successively saves the phasing signals in the number R which are subjected to adaptive phasing processing and are generated by the phasing processing units 204 in the number R for each of the ultrasound wave transmissions. The frame adding unit 702 receives the two or more steering directions from the steering direction instruction unit 112 as synthesis angle information, and reads out the data from the frame memory 701 regarding the designated two or more steering directions, thereby performing adding processing. Accordingly, aperture synthesis processing and data adding in the null direction (adding of the two or more steering directions) can be performed at the same time.

In the eighth embodiment, since the phasing signals obtained through adaptive phasing processing for the steering direction are synthesized, compared to the third embodiment, the effect of decreasing correlative noise resulting from wave-front distortion is significant. In addition, since the null angle can be calculated by using the phasing signal obtained through adaptive phasing processing, there is an effect of achieving high detection accuracy of the null angle as well.

(Ninth Embodiment)

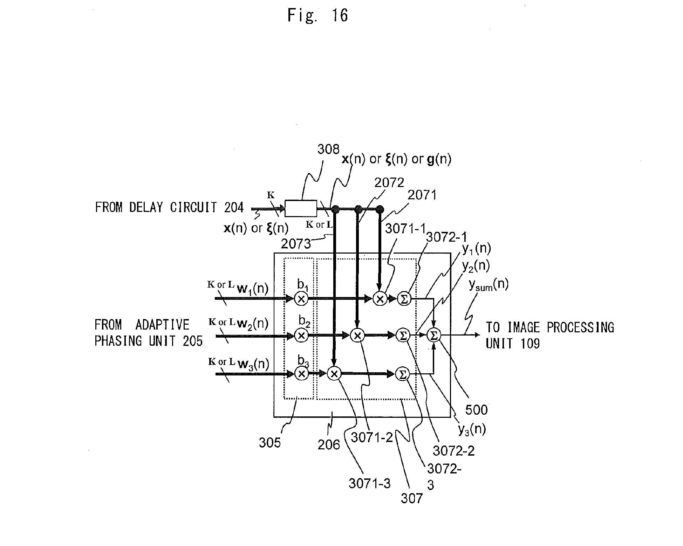

The ultrasound imaging apparatus in a ninth embodiment will be described with reference to Fig. 16.

The ninth embodiment provides another embodiment of the adaptive synthesizing unit different from those in the fifth to eighth embodiments. The inside of the adaptive synthesizing unit in the fifth to eighth embodiments may have the configuration as that in the fifth embodiment illustrated in Fig. 11, or may have the configuration as that of the ninth embodiment in Fig. 16.

In the fifth embodiment illustrated in Fig. 11, the adaptive synthesizing unit 516 is configured to synthesize the multiple adaptive weight vectors $w_1(n)$, $w_2(n)$, and $w_3(n)$ by the weight synthesizing unit 306, and to perform phasing-adding processing of the post-delaying reception data $x(n)$ by the acquired synthesis weight $w_{sum}(n)$. Since this computation is linear-type computation, the order of the weight synthesizing and phasing-adding processing may be inverted. Therefore, in the adaptive synthesizing unit 206 of the ninth embodiment, as illustrated in Fig. 16, the multiple multiplying units (3071-1, 3071-2, and 3071-3) and the multiple adding units 3072-1, 3072-2, and 3072-3 which acquire the pre-synthesizing phasing output by calculating the sum of the post-multiplication elements (post-delaying data) are arranged for each of the multiple adaptive weight vector $w_1(n)$, $w_2(n)$, and $w_3(n)$. A set including each one of the multiplying unit 3071 and the adding unit 3072 configures one inner product computation unit for each adaptive weight vector. The inner product computation unit 307 is configured by preparing the sets in the multiple numbers. The post-delaying reception data $x(n)$ is input to each of the multiplying units 3071-1, 3071-2, and 3071-3 by using bypass inputs 2071, 2072, and 2073. An output synthesizing unit 500 is arranged in the following stage of the inner product computation unit 307.

According to such a configuration, in the inner product computation unit 307, the bypass input 2071 is subjected to phasing-adding by using the weight vector $w_1(n)$. The bypass input 2072 is subjected to phasing-adding by using the weight vector $w_2(n)$, and the bypass input 2073 is subjected to phasing-adding by using the weight vector $w_3(n)$, thereby calculating the pre-synthesizing multiple phasing outputs $y_1(n)$, $y_2(n)$, and $y_3(n)$ corresponding to each of the steering vectors (Expression (23) below). In the following stage thereof, the output synthesizing unit 500 obtains the arithmetic average of the phasing outputs $y_1(n)$, $y_2(n)$, and $y_3(n)$ corresponding to each of the steering vectors, thereby acquiring the synthesizing-phasing output $y_{sum}(n)$ as shown in Expression (24).

[Expression 23]

$$y_p(n) = \mathbf{w}_p^H(n)\mathbf{x}(n) \qquad \cdots (23)$$

[Expression 24]

$$y_{\text{sum}}(n) = \sum_{p=1}^{P} y_p(n) = \frac{1}{P}\left\{ y_1(n) + y_2(n) + \cdots y_p(n) \cdots + y_P(n) \right\} \qquad \cdots (24)$$

As illustrated in Fig. 16, the fixed apodization multiplying unit 305 can be added to the configuration in the present embodiment as well. In Fig. 16, the fixed apodization multiplying unit 305 is arranged in the previous stage of the inner product computation unit 307. However, the position of the fixed apodization multiplying unit 305 is not limited to the position in Fig. 16. As it is clear from the linear-type computation, the fixed apodization multiplying unit 305 may be added between the inner product computation unit 307 and the output synthesizing unit 500. Moreover, the fixed apodization multiplying unit 305 may be added between the multiplying units 3071-1 to 3 and the adding units 3072-1 to 3 in the inner product computation unit. In both cases, the final synthesizing-phasing output $y_{sum}(n)$ can be represented by Expression (25).

[Expression 25]

$$y_{\text{sum}}(n) = \sum_{p=1}^{P} b_p y_p(n) = \frac{1}{P}\left\{ b_1 y_1(n) + b_2 y_2(n) + \cdots b_p y_p(n) \cdots + b_P y_P(n) \right\} \qquad \cdots (25)$$

As described above, the fifth embodiment and the eighth embodiment are different from each other in the embodiment whether inner product computation is performed after the weights are synthesized or the phasing output is synthesized after inner product computation. Meanwhile, the final outputs are identical to each other due to linear computation. Therefore, the values of $y(n)$ in Expression (22) which is the last phasing output is the fifth embodiment and the last synthesizing-phasing output $y_{sum}(n)$ of the eighth embodiment are the same.

(Tenth Embodiment)

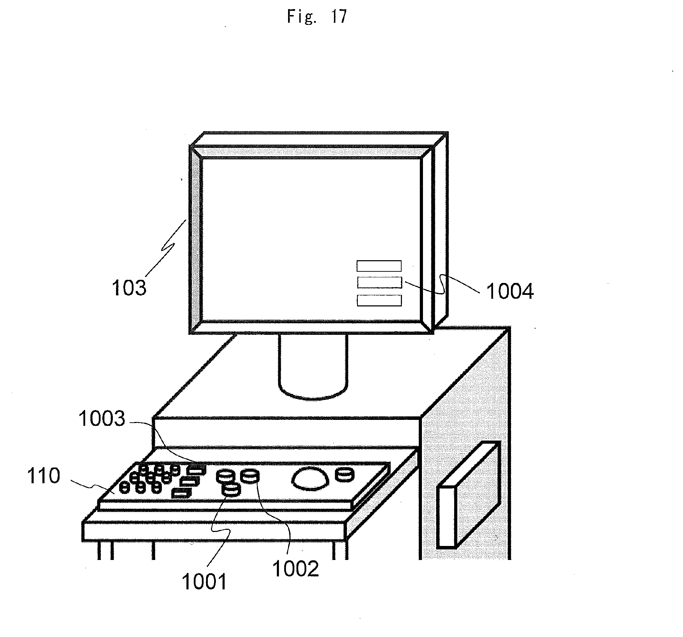

Fig. 17 illustrates an example of the console 110 of the ultrasound imaging apparatus in the first to eighth embodiments. In order to realize the embodiments which have been described above, in the console 110 of the ultrasound diagnostic apparatus, as an operation unit for changing the number of the steering directions and the like, knob portions 1001 and 1002, and the like having a scale can be arranged. In addition, as an operation unit for switching modes between obtaining of the null angle described in each of the embodiments, by performing calculation and detecting of the same, a switch unit 1004 can be arranged in the console 110. Accordingly, an operator can change various parameters in phasing synthesis processing while watching an actual ultrasound image, and can perform imaging and making a diagnosis at an optimal condition form each test object 100. In addition, the set value can be configured to be displayed by a portion 1104 in the display region of the image display unit 103.

In addition, the mode switching unit of the console may be formed so as to be able to switch the mode in accordance with switching in different probes, an imaging condition, and an imaging sequence. For example, it is possible to adopt a switching unit which can switch the mode so as to be able to generate an image by applying a set of the steering directions different from each other, depending on a probe such as a linear probe, a convex probe, a sector probe, a two-dimensional array probe, and a mechanical type 3D imaging probe. In addition, for example, it is possible to adopt a switching unit which can generate an image by applying a set of the steering directions different from each other depending on an imaging condition and an imaging sequence such as transmission/reception focus, a transmission/reception frequency, a frame rate, the number of parallel beamformers, tissue harmonics, and imaging contrast.

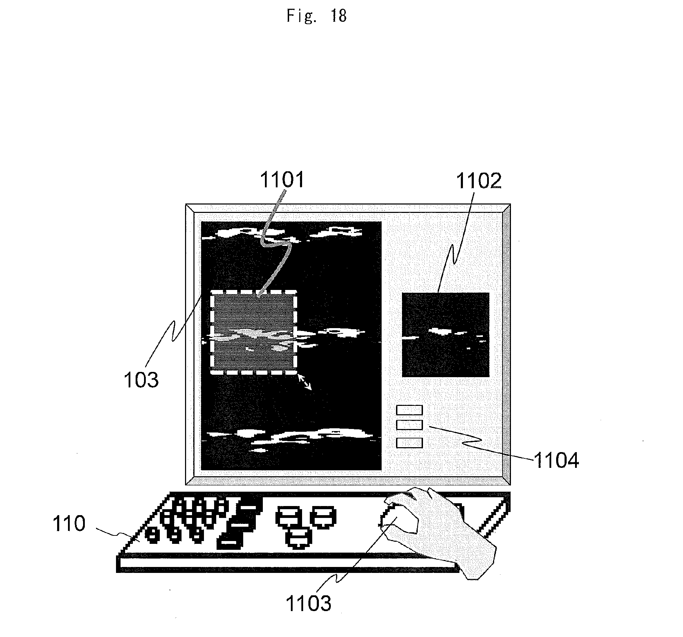

Fig. 18 illustrates a perspective view of the console 110 and the image display unit 103 in another specification example for the ultrasound diagnostic apparatus of the present invention. In the configuration in Fig. 18, while referring to an ordinary ultrasound image (an image not subjected to synthesis processing of the present invention) 103, a practitioner is provided with a handy operation unit 1103 (for example, a mouse) for setting a particular ROI (a region of interest) 1101. Accordingly, the practitioner can generate an image 1102 which is subjected to processing in each embodiment of the present invention, only for the particular ROI 1101. In addition, the image 1102 subjected to processing of the present invention can be displayed in another region in the image display unit 103.

In addition, it is possible to provide a configuration in which a practitioner can set parameters of imaging conditions by using the handy operation unit 1103.

(Specification Example of Effect of Embodiments)

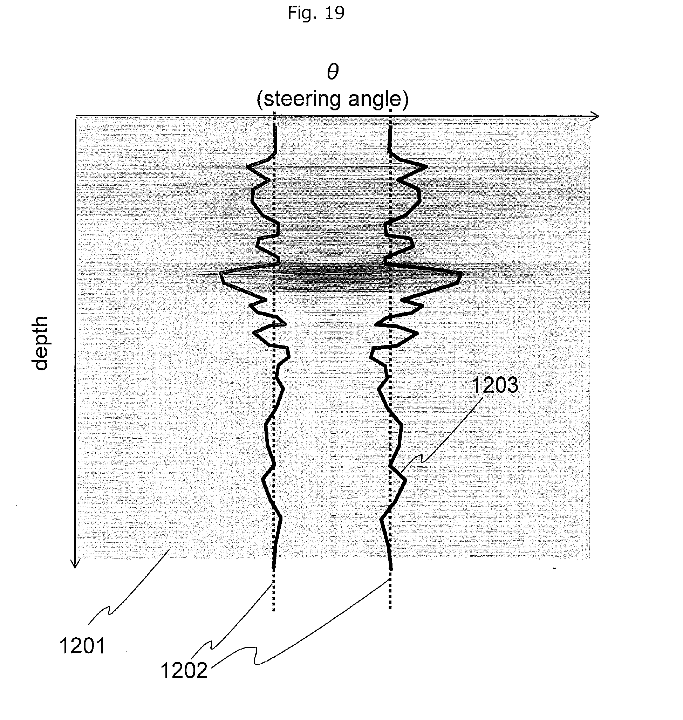

Fig. 19 illustrates a profile of the phasing signal of the phasing processing unit 204 which performs delay adding processing of the fourth embodiment in Fig. 8. Fig. 19 is a two-dimensional map 1201 having the steering angle $\theta$ as the transverse axis and the depth as the vertical axis. A dotted line 1202 in Fig. 19 indicates the null angle of a transmission/reception beam which is calculated by the null angle calculation unit 501 based on the pitch, the frequency, and the like of the ultrasound element 105 according to the configuration of the second embodiment in Fig. 5. A solid line 1203 is a trace of the null angle (the minimal point) detected by the null angle detection unit 207 of the fourth embodiment in Fig. 8 and indicates the null angle in a case where the test object is actually irradiated with an ultrasound wave.

As it is clearly shown in Fig. 19, there is an occurrence of deviation between the null angle decided by performing calculation and the null angle detected by being actually irradiated with an ultrasound wave.

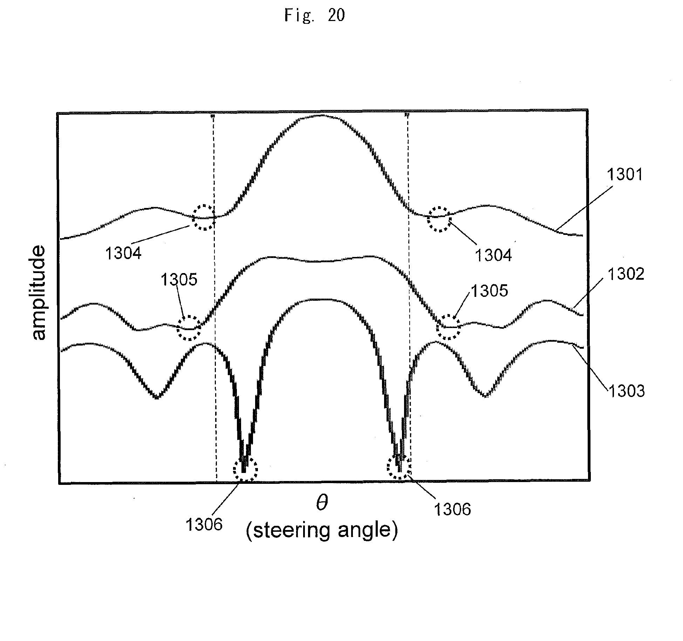

In Fig. 20, magnitude of an output of the phasing signal from the phasing processing unit 204 of the fourth embodiment is illustrated in the steering angle $\theta$ directions regarding three depths in Fig. 19 different from one another. In other words, Fig. 20 illustrates directivity regarding three depths different from one another. As it is clearly shown in Fig. 20, positions 1304, 1305, and 1306 for the minimal values which can be seen on each of the line profiles 1301, 1302, and 1303 are different from one another.

According to Figs. 19 and 20, as shown in the fifth embodiment and the like, by detecting nulls using the null angle detection unit 207, the steering directions can be set to the accurate directions of the null angles. Therefore, it is understood that improvement of an effect of decreasing correlative noise can be expected.

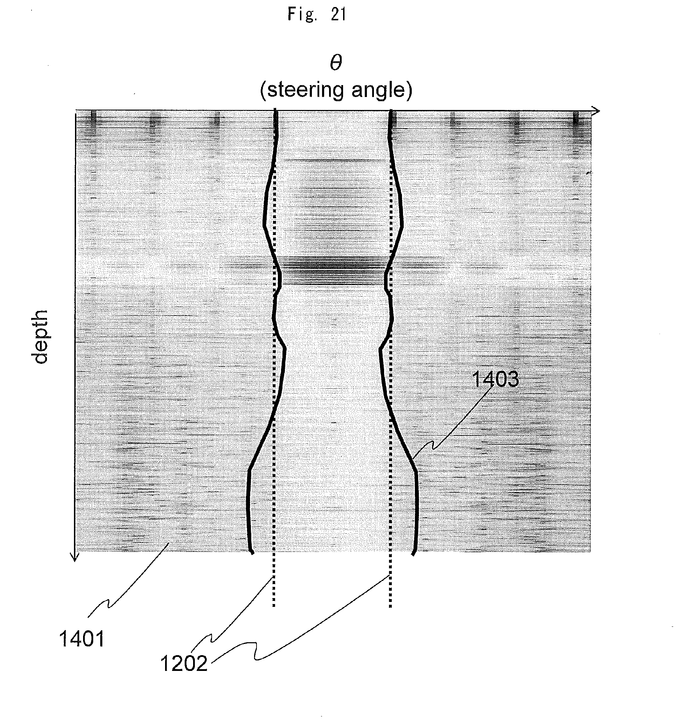

Fig. 21 illustrates a profile of the phasing signal of the phasing processing unit 204 performing adaptive phasing processing of the sixth embodiment in Fig. 21. The dotted line 1202 in Fig. 21 indicates the null angle of a transmission/reception beam which calculated by the null angle calculation unit 501 based on a pitch, a frequency, and the like of the ultrasound element 105 according to the configuration of the second embodiment in Fig. 5. A solid line 1403 is a trace of the null angle (the minimal point) detected by the null angle detection unit 207 of the sixth embodiment in Fig. 12 and indicates the null angle in a case where the test object is actually irradiated with an ultrasound wave.

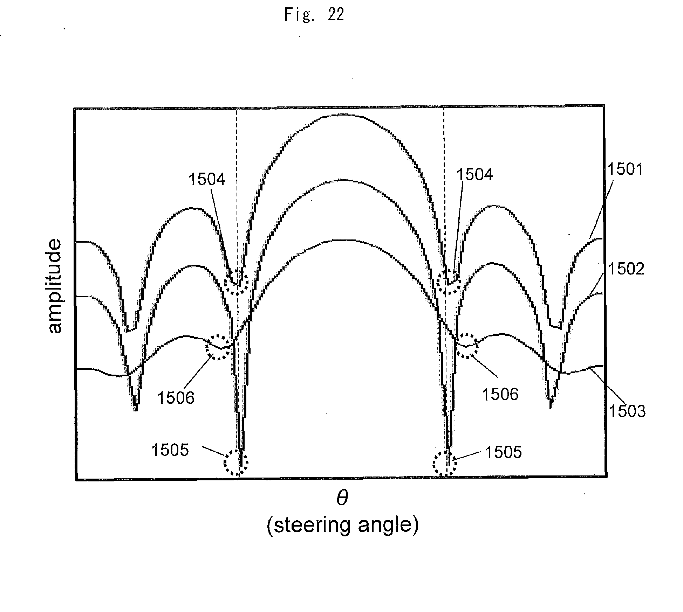

In Fig. 22, magnitude of an output of the phasing signal from the phasing processing unit 204 of the sixth embodiment is illustrated in the steering angle $\theta$ directions regarding three depths in Fig. 21 different from one another. As it is clearly shown in Fig. 22, positions 1504, 1505, and 1506 for the minimal values which can be seen on each of the line profiles 1501, 1502, and 1503 are different from one another.

According to Figs. 21 and 22, in a case of performing adaptive phasing processing, as shown in the sixth embodiment and the like, by detecting nulls using the null angle detection unit 207, the steering directions can be set to the accurate directions of the null angles. Therefore, it is understood that improvement of an effect of decreasing correlative noise can be expected.

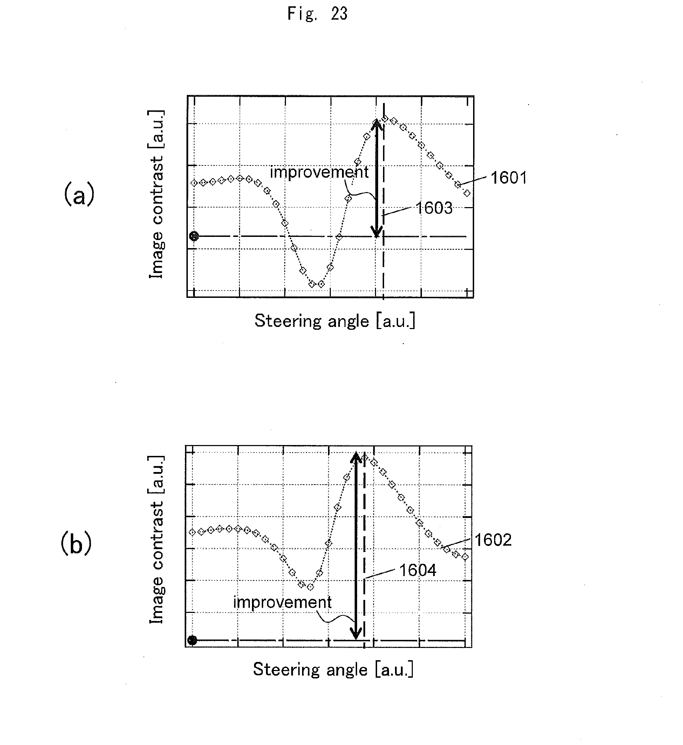

Lastly, with reference to Fig. 23, an effect of null detection according to the present invention will be described. Figs. 23(a) and 23(b) respectively illustrate profiles of the image contrasts 1601 and 1602 regarding the steering directions $\theta$ in certain depths when irradiation is performed with an ultrasound wave with respect to the test targets different from one another by using the ultrasound imaging apparatus in Fig. 12 which detects the null angle through adaptive phasing processing of the sixth embodiment. In Figs. 23(a) and 23(b), each of dotted lines 1603 and 1604 indicates the null angle detected by the null angle detection unit 207. The null angles 1603 and 1604 are different from each other. Accordingly, it is learned that the null angle varies depending on a test object.

As seen in the line profiles 1601 and 1602 in Figs. 23 (a) and 23(b), it is found that the image contrast becomes the maximum at the directions of the null angles. As the image contrast of the null angle, an image contrast which is higher than that in other steering angles by 5 to 6 dB is acquired. According to the above-described contents, the null angle can be detected as described in the sixth embodiment, and the directions of the null angles on the right and left can be set to the steering direction so as to synthesize the phasing signal. Thus, it is possible to decrease noise and to remarkably improve the image contrast.

Reference Signs List

| | |
|---|---|
| 100 | test object |
| 101 | ultrasound element array |
| 102 | apparatus main body |
| 103 | image display unit |
| 104 | transmission beamformer |
| 106 | ultrasound probe |
| 107 | transmission/reception separation circuit (T/R) |
| 108 | reception beamformer |
| 109 | image processing unit |
| 110 | console |
| 111 | control unit |
| 204 | phasing processing unit |
| 205 | memory unit |
| 206 | peripheral information synthesizing unit |
| 207 | null angle detection unit |
| 301 | adaptive beam steering unit |
| 501 | null angle calculation unit |
| 701 | channel memory |
| 702 | frame adding unit |
| 1001, 1002 | knob portion |

## Claims

1. An ultrasound imaging apparatus comprising:

an ultrasound element array that includes multiple ultrasound elements arrayed along a predetermined direction;
a reception beamformer that performs phasing of a signal received by the ultrasound element array; and
an image processing unit that uses an phasing output which is output by the reception beamformer and generates image data,
wherein the reception beamformer includes a phasing synthesizing unit which performs synthesizing after performing phasing processing of the signal received by the ultrasound element array for each of two or more steering directions and a steering direction instruction unit which sets the two or more steering directions to the phasing synthesizing unit, and
wherein the two or more steering directions include at least two directions other than a direction of a reception focal point.

2. The ultrasound imaging apparatus according to Claim 1,
wherein each of the two directions is a direction of a null angle of a directivity profile of the ultrasound element array.

3. The ultrasound imaging apparatus according to Claim 1 or 2,
wherein the two or more steering directions also include the direction of the reception focal point.

4. The ultrasound imaging apparatus according to Claim 2,
wherein the steering direction instruction unit sets preobtained two or more directions of the null angles of the ultrasound element array to the phasing synthesizing unit.

5. The ultrasound imaging apparatus according to Claim 2,
wherein the steering direction instruction unit includes a null angle calculation unit which obtains the two or more directions of the null angles of the ultrasound element array by performing calculation and sets the directions of the null angles which are obtained by the null angle calculation unit to the phasing synthesizing unit.

**6.** The ultrasound imaging apparatus according to Claim 2,
wherein the steering direction instruction unit includes a null angle detection unit which detects two or more null angles from the signal received by the ultrasound element array and sets the directions of the null angles which are obtained by the null angle detection unit to the phasing synthesizing unit.

**7.** The ultrasound imaging apparatus according to Claim 6,
wherein the steering direction instruction unit also includes a null angle calculation unit which obtains the two or more directions of the null angles of the ultrasound element array by performing calculation, and the null angle detection unit detects the null angle within a preset range on the periphery of the null angles obtained by the null angle calculation unit and obtains the null angles.

**8.** The ultrasound imaging apparatus according to Claim 6 or 7,
wherein the phasing synthesizing unit includes a delay adding unit which generates a phasing signal obtained by performing adding after performing delaying of a signal received by each element of the ultrasound element array for each of the multiple steering directions, and the steering direction instruction unit uses the phasing signal output by the delay adding unit and detects a steering direction in which the phasing signal becomes minimal, as the direction of the null angle.

**9.** The ultrasound imaging apparatus according to Claim 6 or 7,
wherein the phasing synthesizing unit includes an adaptive phasing unit which obtains an adaptive weight for each of the two or more steering directions by performing adaptive beam forming and an apodization computation unit which uses the adaptive weight to perform apodization of the signal received by the ultrasound element array, and wherein the steering direction instruction unit detects a steering direction in which a signal output by the apodization computation unit becomes minimal, as the direction of the null angle.

**10.** The ultrasound imaging apparatus according to Claim 1,
wherein the phasing synthesizing unit includes a delay adding unit which generates a phasing signal by performing adding after performing delaying of a signal received by each element of the ultrasound element array in accordance with the steering direction, and a synthesizing unit which synthesizes the phasing signal for each of the two or more steering directions and acquires the phasing output.

**11.** The ultrasound imaging apparatus according to Claim 1,
wherein the phasing synthesizing unit includes an adaptive phasing unit which obtains an adaptive weight for each of the two or more steering directions by performing adaptive beam forming, a weight synthesizing unit which synthesizes the adaptive weights in the two or more steering directions, and an apodization computation unit which uses the adaptive weights synthesized by the weight synthesizing unit to perform synthesizing after performing apodization of the signal received by the ultrasound element array.

**12.** The ultrasound imaging apparatus according to Claim 1,
wherein the phasing synthesizing unit includes an adaptive phasing unit which obtains an adaptive weight for each of the two or more steering directions by performing adaptive beam forming, an apodization computation unit which uses the adaptive weights and performs synthesizing after performing apodization of the signal received by the ultrasound element array, and a synthesizing unit which synthesizes outputs of the apodization computation unit for each of the two or more steering directions.

**13.** The ultrasound imaging apparatus according to Claim 1, further comprising:

a control unit that sequentially sets active channels at positions different from one another in chronological order with respect to the multiple ultrasound elements and transfers a reception signal of the ultrasound element included in the active channel to the reception beamformer,
wherein a point indicated by the two or more steering directions of the active channels at a certain point of time overlaps partially in position with the point indicated by the two or more steering directions of the active channel at a different point of time.

Fig. 1

Fig. 2

Fig. 3

Signal Intensity

$\theta$ Left null-2

$\theta$ Left null-1

0

$\theta$ Right null-1

$\theta$ Right null-2

$\theta$

$S+N_C+N_U$

$S_{Left}+N_{C\_Left}+N_U$

$S_{Rigjht}+N_{C\_Right}+N_U$

$S' \Downarrow + N_C' \Downarrow + N_U' \Downarrow$

Fig. 4

(a)

(b)

Fig. 5

Fig. 6

START

CAUSE MAIN BODY TO OUTPUT CONTROL SIGNAL INDICATING INFORMATION OF CONDITION OF PROBE, IRRADIATION CONDITION OF ULTRASOUND WAVE, SYNTHESIS CONDITION OF NULL BEAM — 61

CAUSE NULL ANGLE CALCULATION UNIT TO RECEIVE INPUT OF CONTROL SIGNAL — 62

CAUSE NULL ANGLE CALCULATION UNIT TO CALCULATE ANGLE TO BE SYNTHESIZED (NULL DIRECTION ANGLE) BASED ON CONTROL SIGNAL, OR TO EXTRACT THE SAME FROM LUT — 63

CAUSE DELAY ADDING UNIT TO RECEIVE INPUT OF CALCULATED/EXTRACTED INFORMATION OF NULL ANGLE — 64

CAUSE DELAY ADDING UNIT TO GENERATE PHASING SIGNAL IN WHICH STEERING ANGLE IS SET FOR EACH OF NULL ANGLE DIRECTION AND FRONT DIRECTION, AND TO OUTPUT GENERATED SIGNAL — 65

CAUSE SYNTHESIZING UNIT TO RECEIVE INPUTS OF PHASING SIGNAL FOR NULL DIRECTION AND FRONT DIRECTION — 66

CAUSE SYNTHESIZING UNIT TO SYNTHESIZE INPUT PHASING SIGNALS FOR NULL DIRECTION AND FRONT DIRECTION, AND TO OUTPUT THE SYNTHESIZED SIGNALS — 67

REPEAT STEPS FOR EVERY SAMPLE

CAUSE IMAGE PROCESSING UNIT TO RECEIVE INPUT OF SYNTHESIS SIGNAL — 68

END

Fig. 7

Fig. 8

Fig. 9

START

CAUSE MAIN BODY TO GENERATE CONTROL SIGNAL OF CONDITION OF PROBE, IRRADIATION CONDITION OF ULTRASOUND WAVE, SYNTHESIS CONDITION OF NULL BEAM, AND TO OUTPUT GENERATED SIGNAL /61

CAUSE DELAY ADDING UNIT TO CALCULATE PHASING OUTPUT CORRESPONDING TO STEERING ANGLE FROM θmin to θmax, AND TO OUTPUT CALCULATED SIGNAL /92

CAUSE NULL ANGLE CALCULATION UNIT TO RECEIVE INPUT OF CONTROL SIGNAL, AND TO CALCULATE NULL ANGLE /62,63

CAUSE MEMORY UNIT TO STORE PHASING OUTPUT CORRESPONDING TO STEERING ANGLE FROM θmin to θmax /93

CAUSE NULL ANGLE DETECTION UNIT TO REQUEST MEMORY UNIT FOR DATA FOR EACH SAMPLE /91

TRANSMITS PHASING OUTPUT FROM θmin to θmax IN RESPONSE TO REQUEST FROM NULL ANGLE DETECTION UNIT /94

CAUSE NULL ANGLE DETECTION UNIT TO RECEIVE PHASING OUTPUT FROM θmin to θmax /95

CAUSE NULL ANGLE DETECTION UNIT TO EXTRACT MINIMUM PHASING OUTPUT AS NULL POSITION FROM ANGLE RANGE $\theta_1$ TO $\theta_2$ WITHIN ANGLE RANGE FROM θmin to θmax, AND TO OUTPUT EXTRACTED SIGNAL (REPEAT STEP AS MANY THE NUMBER SETS FROM $\theta_1$ TO $\theta_2$) /96

CAUSE SYNTHESIS UNIT TO RECEIVE INPUTS OF PHASING SIGNALS FOR NULL DIRECTION AND FRONT DIRECTION /97

REPEAT STEPS FOR EVERY SAMPLE

CAUSE SYNTHESIZING UNIT TO SYNTHESIZE INPUT PHASING SIGNALS OF NULL DIRECTION AND FRONT DIRECTION, AND TO OUTPUT THE SYNTHESIZED SIGNALS /98

REPEAT STEPS FOR EVERY SAMPLE

CAUSE IMAGE PROCESSING UNIT TO RECEIVE INPUT OF SYNTHESIS SIGNAL /99

END

31

Fig. 10

Fig. 11

Fig. 12

Fig. 13

Fig. 14

Fig. 15

Fig. 16

Fig. 17

Fig. 18

Fig. 19

Fig. 20

Fig. 21

θ
(steering angle)

depth

1403

1401

1202

Fig. 22

Fig. 23

(a)

(b)

## INTERNATIONAL SEARCH REPORT

| International application No. |
| --- |
| PCT/JP2014/050344 |

**A. CLASSIFICATION OF SUBJECT MATTER**
*A61B8/00*(2006.01)i

According to International Patent Classification (IPC) or to both national classification and IPC

**B. FIELDS SEARCHED**

Minimum documentation searched (classification system followed by classification symbols)
A61B8/00-8/15, G01N29/00-29/02, 29/04-29/06, 29/09, 29/12-29/26, 29/28-29/30,
29/38, 29/44

Documentation searched other than minimum documentation to the extent that such documents are included in the fields searched
Jitsuyo Shinan Koho          1922-1996   Jitsuyo Shinan Toroku Koho    1996-2014
Kokai Jitsuyo Shinan Koho   1971-2014   Toroku Jitsuyo Shinan Koho   1994-2014

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2011-45708 A (Toshiba Corp.),<br>10 March 2011 (10.03.2011),<br>paragraphs [0029] to [0042]; fig. 4 to 8<br>& US 2012/0078105 A1    & WO 2011/013713 A1<br>& CN 102365054 A | 1-13 |
| A | JP 2009-506683 A (Step Communications Corp.),<br>12 February 2009 (12.02.2009),<br>abstract; paragraphs [0155] to [0157]; fig.<br>17A, B<br>& US 2007/0047743 A1    & EP 1917838 A<br>& WO 2007/025265 A2    & KR 10-2008-0064808 A<br>& CN 101288335 A          & KR 10-0978823 B1 | 1-13 |

☐ Further documents are listed in the continuation of Box C.      ☐ See patent family annex.

| * | Special categories of cited documents: |
| --- | --- |
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    31 January, 2014 (31.01.14) | Date of mailing of the international search report<br>    10 February, 2014 (10.02.14) |
| --- | --- |
| Name and mailing address of the ISA/<br>    Japanese Patent Office | Authorized officer |
| Facsimile No. | Telephone No. |

Form PCT/ISA/210 (second sheet) (July 2009)

46

**EP 2 944 266 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010158374 A